# EUROPEAN PATENT APPLICATION

(11) **EP 4 663 193 A1**
(43) Date of publication of application: **17.12.2025**
(21) Application number: 24753687.3
(22) Date of filing: 13.02.2024
(51) Int. Cl.: A61K 31/575, A61P 19/02, A61P 43/00

(54) **PHARMACEUTICAL COMPOSITION FOR PREVENTION OR TREATMENT OF AGE-RELATED MUSCULOSKELETAL DISORDERS**

(30) Priority: 10.02.2023 KR 20230017744
(71) Applicant: Seoul National University R & DB Foundation, Seoul 08826 (KR)
(72) Inventor: KANG, Chan Hee, Yongin-si, Gyeonggi-do 16850 (KR); KIM, Jin Hong, Seoul 06320 (KR); ROH, Kyeong Hwan, Seoul 08787 (KR); NOH, Jeong Hwan, Seoul 08733 (KR); KANG, Dong Hyun, Seoul 08813 (KR); KIM, Yeon Ju, Seoul 08814 (KR); JANG, Ye Ji, Seoul 08786 (KR)
(74) Representative: dompatent
(86) International application number: PCT/KR2024/002016
(87) International publication number: WO 2024/167366

(57) **Abstract**

The present invention relates to a composition for the prevention, improvement or treatment of age-related musculoskeletal disorders, which includes a lysosomal ABCA1 inhibitor. More particularly, the composition of the present invention inhibits the transport of cellular ABCA1 to lysosomes or suppresses ABCA1 within lysosomes, by including the lysosomal ABCA1 inhibitor, thereby inhibiting lysosomal cholesterol accumulation and the production of senescence-associated secretory phenotype (SASP) factors. Therefore, the composition can inhibit age-related inflammation, and prevent, improve or treat age-related musculoskeletal disorders.

## Description

### [Technical Field]

The present invention relates to a composition for the prevention, improvement or treatment of age-related musculoskeletal disorders, which includes a lysosomal ABCA1 inhibitor.

### [Background Art]

Aging affects joint health due to changes in cartilage and connective tissue. The cartilage within joints becomes thinner, and its components degenerate, thereby reducing the elasticity of the joints and increasing their susceptibility to damage. Consequently, the joint surfaces no longer glide smoothly against each other, and this process may lead to osteoarthritis. In addition, the connective tissue in ligaments and tendons becomes stiffer and weaker, thereby further increasing joint rigidity and vulnerability. These changes limit the range of motion in the joints.

Muscle loss is a process that begins around age 30 and continues throughout life. During this process, muscle tissue mass and the number and size of muscle fibers gradually decrease. Sarcopenia results in a gradual loss of muscle mass and strength. This gradual decrease in muscle strength increases stress on some joints, which may make people more susceptible to arthritis or falls. In addition, certain types of muscle fibers are affected by aging, with the number of fast-twitch muscle fibers decreasing much more than the number of slow-twitch muscle fibers. Therefore, muscles may contract less quickly as people age.

Meanwhile, cholesterol is a type of lipid that combines with triglycerides, low-density lipoprotein (LDL), and high-density lipoprotein (HDL), and is a component of the plasma membrane of cells. It is essential for cell growth, proliferation, and remodeling, and is used to synthesize various hormones and vitamins, making it an indispensable substance for our bodies.

However, if cholesterol is present in excess of the body's requirements or if its components are not balanced, it can penetrate blood vessel walls, trigger various inflammatory reactions, and then clump together to narrow the blood vessels, interfere with proper blood flow, and cause various cardiovascular diseases, which negatively affects health. In particular, recent international statistical analyses and epidemiological studies have confirmed that cholesterol affects cellular senescence and various age-related diseases, but the causal relationship remains unclear.

Above all, it has been discovered that the accumulation of senescent cells is the main cause of various age-related diseases such as cancer, dementia, cardiovascular disease, and degenerative diseases. Accordingly, senescent cell-targeting technology (senotherapy) has received worldwide attention as a next-generation treatment for age-related diseases. However, in order to commercialize the technology, it is necessary to understand the characteristics of senescent cells and develop methods for their regulation.

### [Summary of Invention]

### [Problems to be Solved by Invention]

It is an object of the present invention to provide a pharmaceutical composition for the prevention or treatment of age-related musculoskeletal disorders.

It is another object of the present invention to provide a food composition for the prevention or improvement of age-related musculoskeletal disorders.

It is yet another object of the present invention to provide a method for screening a therapeutic agent for age-related musculoskeletal disorders.

It is still another object of the present invention to provide a composition for diagnosis of age-related musculoskeletal disorders.

### [Means for Solving Problems]

1. A pharmaceutical composition for the prevention or treatment of age-related musculoskeletal disorders, including a lysosomal ABCA1 inhibitor.
2. The pharmaceutical composition according to the above 1, wherein the lysosomal ABCA1 inhibitor includes at least one selected from the group consisting of gRNA, siRNA, shRNA, dsRNA, miRNA, PAN, ribozyme, an antisense nucleic acid, a peptide, a protein, an aptamer, an antibody and a compound.
3. The pharmaceutical composition according to the above 2, wherein the gRNA includes at least one nucleotide sequence selected from the group consisting of SEQ ID NOs: 1 to 4, and the siRNA includes at least one nucleotide sequence selected from the group consisting of SEQ ID NOs: 5 to 8.
4. The pharmaceutical composition according to the above 1, wherein the lysosomal ABCA1 inhibitor is glyburide.
5. The pharmaceutical composition according to the above 1, wherein the lysosomal ABCA1 inhibitor inhibits lysosomal cholesterol accumulation in cells.
6. The pharmaceutical composition according to the above 1, wherein the age-related musculoskeletal disorder is osteoarthritis or sarcopenia.
7. A food composition for the prevention or improvement of age-related musculoskeletal disorders, including a lysosomal ABCA1 inhibitor.
8. The food composition according to the above 7, wherein the lysosomal ABCA1 inhibitor includes at least one selected from the group consisting of gRNA, siRNA, shRNA, dsRNA, miRNA, PAN, ribozyme, an antisense nucleic acid, a peptide, a protein, an aptamer, an antibody and a compound.
9. The food composition according to the above 8, wherein the gRNA includes at least one nucleotide sequence selected from the group consisting of SEQ ID NOs: 1 to 4, and the siRNA includes at least one nucleotide sequence selected from the group consisting of SEQ ID NOs: 5 to 8.
10. The food composition according to the above 7, wherein the lysosomal ABCA1 inhibitor is glyburide.
11. A method for screening a therapeutic agent for age-related musculoskeletal disorders, including treating isolated cells with a candidate substance to determine whether the activity of lysosomal ABCA1 is inhibited.
12. The method according to the above 11, wherein the cells are chondrocytes, fibroblasts or muscle cells.
13. The method according to the above 11, wherein the age-related musculoskeletal disorder is osteoarthritis or sarcopenia.
14. A composition for diagnosis of age-related musculoskeletal disorders, including an agent that specifically binds to lysosomal ABCA1 mRNA or a protein encoded thereby.
15. The composition according to the above 14, wherein the agent is at least one selected from the group consisting of a primer, a probe, an antisense nucleic acid, a peptide, a protein, an aptamer, an antibody and a compound.
16. The composition according to the above 14, wherein the age-related musculoskeletal disorder is osteoarthritis or sarcopenia.

### [Advantageous effects]

The pharmaceutical composition according to the present invention may prevent or treat age-related musculoskeletal disorders by including a lysosomal ABCA1 inhibitor.

The food composition according to the present invention may prevent or alleviate age-related musculoskeletal disorders by including a lysosomal ABCA1 inhibitor.

The screening method of the present invention may identify candidate compounds as therapeutic agents for age-related musculoskeletal disorders by determining whether lysosomal ABCA1 is inhibited.

The diagnostic composition of the present invention may diagnose or predict the prognosis of age-related musculoskeletal disorders by including an agent for measuring the expression level or activity of lysosomal ABCA1.

### [Brief Description of Drawings]

FIG. 1 is a schematic diagram illustrating the activation of age-related inflammation mediated by the lysosomal ABCA1-lysosomal cholesterol-mTORC1/SASP axis.
FIG. 2a is a diagram illustrating the results of increased cholesterol levels in senescence-induced cells. Specifically, the upper panel shows Filipin staining and enzymatic cholesterol measurement results in DNA damage-induced senescent cells (SEN), and the lower panel shows enzymatic cholesterol measurement results for replicative senescent cells (SEN (REP)) and oncogene-induced senescent cells (SEN (OIS)).
FIGS. 2b1 and 2b2 are diagrams illustrating the results of increased cholesterol transporter gene mRNA levels in senescence-induced cells, and specifically showing qRT-PCR results of cholesterol transporter gene mRNA in replicative senescent cells (SEN (REP)) and oncogene-induced senescent cells (SEN (OIS)).
FIG. 3a is a diagram illustrating the results of decreased cholesterol levels and reduced SASP factors under cholesterol-depleted (MβCD+) conditions in senescence-induced cells, increased SASP factors under cholesterol-stimulated (Chol+) conditions, with no significant effect on cell growth arrest.
FIG. 3b is a diagram illustrating the results of decreased expression of SASP factors and the cholesterol transporter gene ABCA1, which were increased in senescence-induced cells, by treatment with lovastatin (Lov), a de novo cholesterol synthesis inhibitor.
FIGS. 4a and 4b are diagrams illustrating the results of decreased SASP factors under ABCA1 knockout conditions in senescence-induced cells, with no significant effect on cell growth arrest.
FIG. 4c is a diagram illustrating the results of increased SASP factors, which were reduced in ABCA1 knockout cells, by partial activation of ABCA1.
FIG. 4d is a diagram illustrating that cholesterol stimulation (Chol+) had no affect on the expression of SASP factors in ABCA1 knockout cells.
FIG. 5a is a diagram illustrating that the expression of ABCA1 was increased by ectopic expression of GATA4 (left), and that the expression of ABCA1 and SASP factors, which were increased in senescence-induced cells (SEN), was reduced by GATA4 deletion or NF-κB inhibition (right).
FIG. 5b is a diagram illustrating that GATA4 and endogenous RELA, a major NF-κB subunit, directly bind to the promoter region of ABCA1.
FIG. 6 is a diagram illustrating the correlation between ABCA1 and mTORC1 pathway activity. Specifically, FIG. 6a illustrates changes in mTORC1 signaling activity upon senescence induction (SEN) and ABCA1 deletion, and FIG. 6b illustrates the results of phosphorylation of the mTORC1 substrate S6-kinase 1 (S6K1) and the global translation rate (AFU) upon ABCA1 deletion.
FIGS. 7a and 7b are diagrams illustrating the abundant lysosomal expression of ABCA1, as determined by immunoblotting of lysosome immunoprecipitates (LysoIP).
FIG. 7c is a diagram illustrating the reduced accumulation of lysosomal cholesterol in senescence-induced cells lacking ABCA1. Specifically, the left panel shows staining with Filipin and LysoTracker Red (Lyso); the middle panel shows Pearson's correlation coefficient for the co-localization of cellular cholesterol and lysosomes; and the right panel shows enzymatic cholesterol measurement results.
FIG. 7d is a diagram illustrating the results of increased accumulation of lysosomal cholesterol upon GATA4 expression induction. Specifically, the left panel shows staining with Filipin and LysoTracker Red (Lyso), and the right panel shows Pearson's correlation coefficient for the co-localization of cellular cholesterol and lysosomes.
FIG. 8a is a diagram illustrating the results of enhanced expression of lysosomal ABCA1, which was increased in senescence-induced cells, by probucol treatment. Specifically, the left panel shows immunoblotting results of lysosome immunoprecipitates (LysoIP), and the right panel shows enzymatic cholesterol measurement results.
FIG. 8b is a diagram illustrating mRNA qRT-PCR results showing that the expression of SASP factors in senescence-induced cells was further increased by probucol treatment, with minimal effect on the expression of CDKN2A or CDKN1A.
FIG. 8c is a diagram illustrating that SASP activity was increased by probucol treatment in senescence-induced cells, whereas probucol treatment had no significant effect on SASP activation in senescence-induced cells lacking ABCA1.
FIG. 8d is a diagram illustrating that SASP production was increased through mTORC1 activation by probucol treatment in senescence-induced cells.
FIG. 8e is a diagram illustrating that SASP activation induced by probucol treatment was suppressed by the mTORC1 inhibitor rapamycin.
FIGS. 8f1 and 8f2 are diagrams illustrating that lysosomal cholesterol accumulation and SASP activity were suppressed by glyburide treatment in senescence-induced cells.
FIG. 8g is a diagram illustrating that SASP factor expression, which was activated by probucol, was suppressed by glyburide treatment in senescence-induced cells treated with probucol.
FIG. 8h is a diagram illustrating that total cellular cholesterol levels were increased, while lysosomal cholesterol levels were decreased by genetic deletion of ABCA1.
FIG. 9a is a diagram illustrating that lysosomal microdomain formation (FLOTILLIN-1 positive) and the enrichment of mTORC1 scaffolding complex , including Ragulator, SLC38A9 and V-ATPase, were observed in senescence-induced cells, whereas lysosomal microdomain formation and mTORC1 scaffolding complex activity were reduced in senescence-induced cells lacking ABCA1.
FIGS. 9b1 and 9b2 are diagrams illustrating that the lysosomal localization of mTORC1 is persistently decreased and that lysosomal cholesterol accumulation is reduced in senescence-induced cells lacking ABCA1, as determined by immunofluorescence analysis.
FIG. 10a is a diagram illustrating the GSEA results showing that enrichment scores for cholesterol homeostasis and mTORC1 pathway signatures were significantly upregulated in genes that were differentially expressed during osteoarthritis progression.
FIG. 10b is a diagram illustrating the positive correlation between ABCA1 and SASP factors, including MMP13, IL1B, IL8 and CCL8.
FIGS.11a1 and 11a2 are diagrams illustrating that genes such as Scarb1 and Abca1 were significantly upregulated, and cholesterol levels were increased during senescence of chondrocytes.
FIG. 11b is a diagram illustrating that the expression of SASP factors, including Mmp3, Mmp13 and Cxcl1, was suppressed in mouse primary cultured chondrocytes (SEN) with induced ABCA1 deletion.
FIG. 11c is a diagram illustrating that SASP factors were upregulated upon probucol treatment and were suppressed upon treatment with glyburide or lovastatin in mouse primary cultured chondrocytes (SEN) with induced ABCA1 deletion.
FIG. 11d is a diagram illustrating that the sustained activation pattern of mTORC1 was suppressed upon cholesterol depletion induction in mouse primary cultured chondrocytes (SEN).
FIGS. 12a to 12c are diagrams illustrating that the progression of cartilage damage was alleviated, senescence markers such as SASP factors, p16 and nuclear HMGB1 were reduced, and osteoarthritis-related pain indicators were also alleviated, as a result of glyburide administration in a mouse model of osteoarthritis progression.
FIGS. 13a and 13b are diagrams illustrating a positive correlation between the SASP signature score and the expression of ABCA1 in various tissues of elderly subjects, including the brain and lungs outside the joints.

### [Mode for Carrying out Invention]

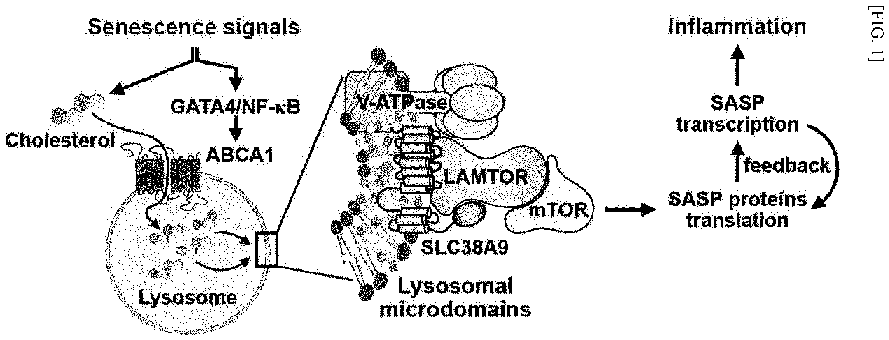

The present inventors have identified the mechanism of action in which changes in cholesterol metabolism in the aging mechanism are due to the shift of the intracellular location of the cholesterol efflux regulator ABCA1 from the cell membrane to the lysosome. Specifically, the present inventors have elucidated the mechanism by which the expression of ABCA1 in the lysosomes of cells in osteoarthritis tissue leads to lysosomal cholesterol accumulation, which in turn induces structural alterations in the lysosome, resulting in the upregulation of mTORC1, a key regulator of nutrient and energy metabolism. The upregulation of mTORC1 results in abnormal activation of senescent cells, thereby promoting the production of senescence-associated secretory phenotype (SASP) factors, which trigger chronic inflammation, accelerate aging, and worsen pathological conditions (FIG. 1).

The present invention relates, in one aspect, to a pharmaceutical composition for the prevention or treatment of age-related musculoskeletal disorders, which includes a lysosomal ABCA1 inhibitor.

ATP-binding cassette transporter ABCA1 (ATP-binding cassette subfamily A member 1), also known as cholesterol efflux regulatory protein (CERP), is a protein encoded by the ABCA1 gene. This transporter is a major regulator of cellular cholesterol and phospholipid homeostasis.

As used herein, the term "lysosomal ABCA1 inhibitor" may refer to an agent that directly inhibits lysosomal ABCA1. The direct inhibition of lysosomal ABCA1 may refer to inhibition of the activity of ABCA1 present in or localized to lysosomes, or preventing the intracellular localization of ABCA1 present in cells from being re-routed and transported to lysosomes.

Therefore, the direct inhibitor of lysosomal ABCA1 includes all agents that inhibit or suppress the activity of ABCA1 protein localized in lysosomes of cells. In addition, the inhibitor includes all agents that inhibit or suppress the expression of the gene encoding ABCA1 protein localized in the lysosomes of cells. Further, the inhibitor includes all agents that inhibit the transport of intracellular ABCA1 to lysosomes.

In the present invention, the lysosomal ABCA1 inhibitor may include at least one selected from the group consisting of gRNA, siRNA, shRNA, dsRNA, miRNA, PAN, ribozyme, an antisense nucleic acid, a peptide, a protein, an aptamer, an antibody and a compound.

More specifically, any substance that inhibits the transport of intracellular ABCA1 to lysosomes or inhibits the activity of ABCA1 in lysosomes may be used, without limitation in form, as a compound, nucleic acid, peptide, virus, or vector including a nucleic acid targeting lysosomal ABCA1.

The expression 'targeting lysosomal ABCA1' refers to targeting ABCA1 that is transported to lysosomes within cells or ABCA1 present in lysosomes, and hereinafter, 'lysosomal ABCA1' shall be interpreted as having this meaning.

The lysosomal ABCA1 inhibitor of the present invention may directly inhibit lysosomal ABCA1 by targeting lysosomal ABCA1.

In the present invention, the lysosomal ABCA1 inhibitor is not limited in form, and may include a compound, peptide, peptidomimetic, substrate analog, antibody or aptamer that reduces the activity of lysosomal ABCA1 protein.

In the present invention, the aptamer refers to a nucleic acid molecule having binding affinity to a specific target molecule. The aptamer may have various three-dimensional structures depending on its base sequence, and may have high affinity for a specific substance, similar to an antigen-antibody reaction. The aptamer may inhibit the activity of a specific target molecule by binding to the specific target molecule. The aptamer may be RNA, DNA, a modified nucleic acid, or a mixture thereof, and may be linear or circular, but is not limited thereto. The aptamer that inhibits the activity of ABCA1 of the present invention may be easily produced by a person having ordinary skill in the art by referring to the base sequence of ABCA1 using a known method.

In the present invention, the compound is included in the scope of the present invention without limitation as long as it is a compound that inhibits the activity of lysosomal ABCA1, and may be used without limitation in the form of a drug, either alone or in combination with other compounds.

For example, the drug may be glyburide, but is not limited thereto.

In an embodiment of the present invention, the progression of osteoarthritis was compared between groups treated with two drugs (Probucol and Glyburide), both affecting ABCA1 expression, using a male mouse model subjected to destabilization of the medial meniscus (DMM) surgery. As a result, it was confirmed that Probucol treatment increased lysosomal ABCA1 and promoted lysosomal cholesterol accumulation, thereby worsening osteoarthritis progression and pain, whereas Glyburide treatment suppressed the increase in lysosomal ABCA1 and inhibited lysosomal cholesterol accumulation, thereby alleviating osteoarthritis progression and pain.

Probucol is a drug that has been reported to limit the intracellular localization of ABCA1 to intracellular compartments, including lysosomes. The results of the embodiment confirm that the increase in lysosomal ABCA1 following probucol treatment results from a change in the intracellular localization of ABCA1 to lysosomes induced by probucol. On the other hand, the decrease in lysosomal ABCA1 following glyburide treatment results from glyburide inhibiting the transport of ABCA1 to lysosomes within cells.

With these results, the inventors of the present invention have identified that ABCA1 is not merely involved in regulating total cholesterol levels as a cellular cholesterol transporter, but also plays a direct role in regulating lysosomal cholesterol. They have further confirmed that directly targeting lysosomal ABCA1 levels is effective in suppressing age-related inflammation and treating musculoskeletal disorders associated with aging.

In the present invention, an inhibitor that reduces the expression of the ABCA1 gene encoding the lysosomal ABCA1 protein may be used without limitation in form, such as gRNA, siRNA, shRNA, dsRNA, miRNA, PAN, ribozyme, or antisense nucleic acid.

For example, the inhibitor may include an antisense oligonucleotide (ASO), guide RNA (gRNA), small hairpin RNA (shRNA), small interfering RNA (siRNA), or micro RNA (miRNA) that specifically targets the gene encoding lysosomal ABCA1.

In the present invention, the gRNA may include at least one nucleotide sequence selected from the group consisting of SEQ ID NOs: 1 to 4.

The gRNA refers to a ribonucleic acid that is complementary to a sequence selected from SEQ ID NOs: 1 to 4 or a corresponding sequence, and serves to guide an endonuclease protein to a target DNA sequence by binding to the target DNA sequence in full or partial complementarity. The gRNA refers to a dual RNA composed of two RNAs, namely CRISPR RNA (crRNA) and trans-activating crRNA (tracrRNA); or a single-stranded guide RNA (sgRNA) that includes a first portion including a sequence complementary in whole or in part to a sequence in the target DNA and a second portion including a sequence that interacts with an RNA-guided nuclease. However, any form of RNA that enables an RNA-guided endonuclease to be active at the target DNA sequence may fall within the scope of the present invention without limitation. The gRNA according to the present invention is preferably in the form of a single-stranded guide RNA (sgRNA), but is not limited thereto, and may be appropriately selected depending on the type of endonuclease used or the microorganism from which it is derived.

In an embodiment of the present invention, a cell clone to which a vector expressing a gRNA including at least one nucleotide sequence selected from the group consisting of SEQ ID NOs: 1 to 4 was delivered exhibited inhibition of lysosomal ABCA1, resulting in suppression of lysosomal cholesterol accumulation, inhibition of mTORC1 activity, and reduced SASP production, thereby demonstrating an effect of suppressing age-related inflammation.

In the present invention, the gRNA may further include a base sequence represented by SEQ ID NO: 9. The gRNA represented by SEQ ID NO: 9 specifically targets the gene encoding GATA4, thereby knocking out GATA4, and the knockout of GATA4 may promote the inhibition of ABCA1.

In the present invention, siRNA refers to a nucleic acid molecule capable of inducing RNA interference or gene silencing. The length of the siRNA is not particularly limited, and may be, for example, 19 to 25 nucleotides. siRNA may be used as an efficient method for gene knockdown or gene therapy because it can inhibit the expression of a target gene. siRNA may be produced by cleavage of doublestranded RNA by Dicer and may specifically bind to mRNA having a complementary sequence to inhibit the expression of the corresponding mRNA. The siRNA of the present invention may be used to inhibit the expression of ABCA1.

In an embodiment of the present invention, the siRNA may include at least one nucleotide sequence selected from the group consisting of SEQ ID NOs: 5 to 8.

In an embodiment of the present invention, mouse primary cultured chondrocytes, in which ABCA1 was depleted by siRNA including at least one nucleotide sequence selected from the group consisting of SEQ ID NOs: 5 to 8 exhibited suppressed lysosomal ABCA1, thereby inhibiting lysosomal cholesterol accumulation, mTORC1 activity, and SASP production.

In the present invention, the lysosomal ABCA1 inhibitor may inhibit the production of SASP, an age-related inflammatory factor of cells, and suppress age-related inflammation by suppressing lysosomal cholesterol accumulation in cells.

In the present invention, the age-related musculoskeletal disorder may be osteoarthritis or sarcopenia, but is not limited thereto.

The pharmaceutical composition of the present invention may include a pharmaceutically effective amount of a lysosomal ABCA1 inhibitor as an active ingredient.

In addition, the pharmaceutical composition of the present invention may include a pharmaceutically acceptable salt of the lysosomal ABCA1 inhibitor as an active ingredient. The term "pharmaceutically acceptable salt" refers to a salt derived from a pharmaceutically acceptable inorganic acid, organic acid, or base.

Further, the pharmaceutical composition of the present invention may further include an agent that provides an additional lysosomal ABCA1 inhibitory function or therapeutic effect for age-related musculoskeletal disorders in addition to the pharmaceutically effective amount of the lysosomal ABCA1 inhibitor. For example, by further including hyaluronic acid, the quality of synovial fluid may be enhanced, and joint pain may be relieved.

The content of the lysosomal ABCA1 inhibitor in the pharmaceutical composition of the present invention may be appropriately adjusted depending on the symptoms of the disease, the degree of progression of the symptoms, the condition of the patient, etc., and may be, for example, 0.0001 to 99.9% by weight ("wt%") or 0.001 to 50 wt% based on the total weight of the composition, but is not limited thereto. The content ratio is a value based on the dry weight after removal of the solvent.

The pharmaceutical composition of the present invention may be prepared using pharmaceutically acceptable and physiologically suitable additives. As the additive, for example, excipients, disintegrants, sweeteners, binders, coating agents, swelling agents, lubricants, glidants, or flavoring agents may be used.

Further, the pharmaceutical composition of the present invention is preferably formulated as a pharmaceutical composition that includes at least one pharmaceutically acceptable carrier in addition to the active ingredient in a pharmaceutically effective amount for administration, as described above.

The "pharmaceutically effective amount" means an amount sufficient to treat a disease with a reasonable benefit/risk ratio applicable to medical treatment, and an effective dose level may be determined based on factors including the type and severity of the patient's disease, the activity of the drug, the sensitivity to the drug, the time of administration, the route of administration and excretion rate, the duration of treatment, co-administered drugs, and other factors well known in the medical field. The pharmaceutical composition according to the present invention may be administered as an individual therapeutic agent or administered in combination with other therapeutic agents. Further, the composition may be administered sequentially or simultaneously with a conventional therapeutic agent, and may be administered in a single dose or multiple doses. In consideration of all of the above factors, it is important to administer the minimum amount capable of achieving the maximum effect without side effects, which can be easily determined by those skilled in the art.

Specifically, the effective amount of the pharmaceutical composition according to the present invention may vary depending on the age, gender, condition, or body weight of the patient; the absorption, inactivation and excretion rate of the active ingredient in the body; type of disease; and co-administered drugs. Typically, 0.001 to 150 mg, preferably 0.01 to 100 mg, per 1 kg of body weight may be administered once daily, every other day, or divided into one or more doses per day. However, the dosage may be increased or decreased depending on the route of administration, severity of obesity, gender, body weight, or age, etc., and therefore, the above dosage shall not be construed as limiting the scope of the present invention in any way.

Examples of the carrier, excipient and diluent may include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oils. In addition, the composition may further include fillers, anti-aggregating agents, lubricants, wetting agents, flavoring agents, emulsifying agents, and preservatives.

Further, the composition of the present invention may be formulated using methods well known in the art to provide immediate, sustained, or delayed release of the active ingredient after administration to a subject in need of the pharmaceutical composition of the present invention, including humans. The formulation may be in the form of a powder, granule, tablet, emulsion, syrup, aerosol, soft or hard gelatin capsule, sterile injectable solution, or sterile powder.

In addition, the present invention relates to a method for treating age-related musculoskeletal disorders, which includes the step of administering a lysosomal ABCA1 inhibitor to a subject.

The subject is an individual suspected of having age-related musculoskeletal disorders or diagnosed with age-related musculoskeletal disorders, and refers to mammals such as horses, sheep, monkeys, pigs, goats, and dogs, including humans, but is preferably a human.

In another aspect, the present invention relates to a food composition for the prevention or improvement of age-related musculoskeletal disorders, which includes a lysosomal ABCA1 inhibitor.

The lysosomal ABCA1 inhibitor is as described above.

The food composition of the present invention may include conventional food additives in addition to the lysosomal ABCA1 inhibitor or its granules. The suitability of the food additives shall be determined in accordance with the standards and regulations for the relevant items, based on the general principles and test methods of the food additive regulations approved by the U.S. Food and Drug Administration (FDA), unless otherwise specified.

Items listed in the Food Additives Code include, for example, chemical compounds such as ketones, glycine, calcium citrate, nicotinic acid, and cinnamic acid; natural additives such as persimmon pigment, licorice extract, crystalline cellulose, millet pigment, and guar gum; and mixed preparations such as sodium L-glutamate preparations, alkali agents for noodles, preservatives, and tar color preparations, but are not limited thereto.

For example, a health functional food in tablet form may be prepared by granulating a mixture of the lysosomal ABCA1 inhibitor with an excipient, a binder, a disintegrant and other additives in a conventional manner, which is then compression-molded with addition of a lubricant, or by directly compression-molding the mixture. Further, the health functional food in the tablet form may contain a flavoring agent, if necessary.

Health functional foods in the form of capsules may include hard capsules, which can be prepared by filling a mixture of the lysosomal ABCA1 inhibitor and additives such as excipients into a conventional hard capsule, while a soft capsule agent may be prepared by filling a mixture of the lysosomal ABCA1 inhibitor and additives such as excipients into a capsule base such as gelatin. The soft capsule agent may further contain a plasticizer such as glycerin or sorbitol, a colorant, a preservative, and the like, if necessary.

A health functional food in the form of pills may be prepared by molding a mixture of the lysosomal ABCA1 inhibitor, an excipient, a binder, a disintegrant, and other additives using any existing method known in the art. Further, if necessary, it may be coated with sucrose or other coating agents, or may be surface-coated with a material such as starch, talc, etc.

A health functional food in the form of granules may be prepared by granulating a mixture of the lysosomal ABCA1 inhibitor and additives such as an excipient, a binder, and a disintegrant, using any existing method known in the art, and may contain a fragrance agent or a flavoring agent, etc. if necessary.

The health functional food may include, for example, beverages, meats, chocolates, general food products, confectionery, pizzas, ramens, other noodles, gums, candies, ice creams, alcoholic beverages, vitamin complexes, and health supplements.

The health functional food may be administered orally as a nutritional supplement, and the dosage form is not particularly limited. For example, in the case of oral administration, the daily intake is preferably 5,000 mg or less, more preferably 2,000 mg or less, and most preferably 1,000 mg or less. When formulated as a capsule or tablet, one tablet may be taken once a day with water.

In yet another aspect, the present invention relates to a method for screening a therapeutic agent for age-related musculoskeletal disorders, which includes the step of treating isolated cells with a candidate substance to determine whether the activity of lysosomal ABCA1 is inhibited.

The cells may be chondrocytes, fibroblasts or muscle cells, but are not limited thereto.

The chondrocytes may be, for example, mouse primary cultured chondrocytes, primary cultured chondrocytes derived from patients with osteoarthritis, the SW1353 cell line, human chondrocyte cell lines including C20A4, TC28A2 or C28I2, H4 mouse chondrocytes (RRID: CVCL_W634), mouse chondrocyte cell lines including ATDC5 or MC615, etc.

The fibroblasts may be, for example, mouse primary cultured fibroblasts, human primary cultured fibroblasts, mouse fibroblast cell lines NIH/3T3, SV40 MEF, GPE86, or DR4 MEF, and human fibroblast cell lines IMR90, BJ, or NHDF, etc.

The muscle cells may be, for example, mouse primary cultured muscle cells, human primary cultured muscle cells, human muscle cell line AC16, mouse primary cultured myoblasts, human primary cultured myoblasts, mouse myoblast cell line C2C12, or human myoblast cell line LHCN-M2, etc.

Since the method analyzes the improvement of age-related musculoskeletal disorders when the candidate substance administration reduces the level of lysosomal ABCA1 mRNA or a protein encoded thereby, such a candidate substance may be screened as a potential preventive or therapeutic agent for age-related musculoskeletal disorders.

In addition, the method may involve treating a sample isolated from an individual with an age-related musculoskeletal disorder with the candidate substance and comparing the expression level or activity of lysosomal ABCA1 mRNA or protein before and after treatment, or may involve treating only some individuals among multiple subjects with the candidate substance and comparing the expression level with that of the control.

The candidate substance may include, without limitation, a newly synthesized or known substance that is expected to be effective in preventing or treating age-related musculoskeletal disorders. The candidate substance also refers to an unknown compound used in screening to determine whether it affects the expression level of the mRNA or protein of the present invention. The candidate substance may include a chemical substance, a nucleotide, an antisense RNA, a small interfering RNA (siRNA), or a natural product extract, but is not limited thereto.

The age-related musculoskeletal disorder may be osteoarthritis or sarcopenia, but is not limited thereto. In still another aspect, the present invention relates to a composition for diagnosis of age-related musculoskeletal disorders, which includes an agent that specifically binds to lysosomal ABCA1 mRNA or a protein encoded thereby.

Lysosomal ABCA1 is present in a sample derived from an individual, and the available mRNA or protein sequences may be based on sequences known in the NCBI Genbank or the like.

The individual may be an animal that currently has an age-related musculoskeletal disorder, has a history of an age-related musculoskeletal disorder, is suspected of having an age-related musculoskeletal disorder, or is an animal from which diagnostic information regarding other age-related musculoskeletal disorders is desired. The animal may be a mammal including a human. The age-related musculoskeletal disorder may be osteoarthritis or sarcopenia, but is not limited thereto.

The sample is isolated from an individual, and examples thereof may include tissue, cells, blood, serum, plasma, saliva, synovial fluid, or urine, and is preferably a cell sample, but is not limited thereto.

The agent may specifically bind to lysosomal ABCA1 mRNA or a protein encoded thereby in a cell. The agent may be a substance that detects lysosomal ABCA1 mRNA or a protein encoded thereby in a sample isolated from an individual.

The agent is not particularly limited as long as it can detect lysosomal ABCA1 mRNA or a protein encoded thereby, but may be at least one selected from the group consisting of a primer, a probe, an antisense nucleic acid, a peptide, a protein, an aptamer, an antibody and a compound, for example.

The presence or onset of an age-related musculoskeletal disorder may be diagnosed by treating a sample isolated from an individual with the composition of the present invention, and measuring the expression level or activity of lysosomal ABCA1 mRNA or a protein encoded thereby in the sample. For example, by comparing the expression level of lysosomal ABCA1 mRNA or protein in a sample from an individual suspected of having the disorder with that of the control (non-suspected individuals), if the expression level in the suspected individual is found to be higher, the risk or likelihood of developing an age-related musculoskeletal disorder may be determined to be higher. Alternatively, by comparing the expression levels of lysosomal ABCA1 mRNA or protein in samples from two suspected individuals, the individual with the higher expression level may be determined to have a higher risk of developing the disorder or to be at a more advanced stage of the disease.

In addition, the cut-off value may be derived using methods known in the art to determine the presence or onset of an age-related musculoskeletal disorder. For example, by obtaining data on the expression level or activity of lysosomal ABCA1 mRNA or protein in a group of patients with age-related musculoskeletal disorders and in a non-patient group, a cut-off value that distinguishes the patient group from the non-patient group may be established. If the expression level or activity of lysosomal ABCA1 mRNA or protein in a sample from an individual exceeds the cut-off, the individual may be diagnosed as having developed an age-related musculoskeletal disorder, but is not limited thereto.

The present invention may provide a kit for the diagnosis of age-related musculoskeletal disorders, which includes the composition for diagnosis of age-related musculoskeletal disorders.

The kit may diagnose an age-related musculoskeletal disorder by measuring the expression level or activity of lysosomal ABCA1 mRNA or a protein encoded thereby. The diagnostic method and criteria are as described above.

### [Embodiments for Carrying out Invention]

Hereinafter, the present invention will be described in more detail through examples.

### Preparation of aging model and osteoarthritis model

DNA damage-induced senescence was induced in human diploid fibroblasts (IMR90, BJ) by treatment with ionizing radiation (12 Gy) or bleomycin (10 µg/ml, for 24 hours). Specifically, IMR90 and BJ cells expressing hTERT (DNA damage-induced senescence; SEN), IMR90 cells co-expressing hTERT and H-RasV12 [oncogene-induced senescence; SEN (OIS)], and replication-exhausted [replicative senescent cells; SEN (REP)] IMR90 cells were prepared. Normal proliferating cells (PRO) were used as the control.

Mouse primary cultured chondrocytes were isolated from the femoral condyle and tibial plateau of 5-day-old ICR mice.

In the case of mice, male (C57BL/6J) mice were used since estrogen in female mice may influence osteoarthritis progression and serve as a confounding factor. Post-traumatic osteoarthritis was induced in 12-week-old male mice by destabilization of the medial meniscus (DMM) surgery.

The *in vitro* cell model lacking ABCA1 was prepared using the LentiCRISPR-V2 vector and gRNAs of SEQ ID NOs: 1 to 4 below.
ABCA1 gRNA#1: SEQ ID NO: 1
ABCA1 gRNA#2: SEQ ID NO: 2
ABCA1 gRNA#3: SEQ ID NO: 3
ABCA1 gRNA#4: SEQ ID NO: 4
The ABCA1-deficient mouse primary chondrocyte model was prepared using siRNAs of SEQ ID NOs: 5 to 8.
ABCA1 siRNA#1: SEQ ID NO. 5
ABCA1 siRNA#2: SEQ ID NO. 6
ABCA1 siRNA#3: SEQ ID NO. 7
ABCA1 siRNA#4: SEQ ID NO. 8

### Example 1. Changes in cholesterol metabolism during senescence

### 1-1. Increase in cholesterol transporter genes during senescence

To confirm the mechanism of cholesterol-induced aging, changes in intracellular cholesterol levels were directly assessed.

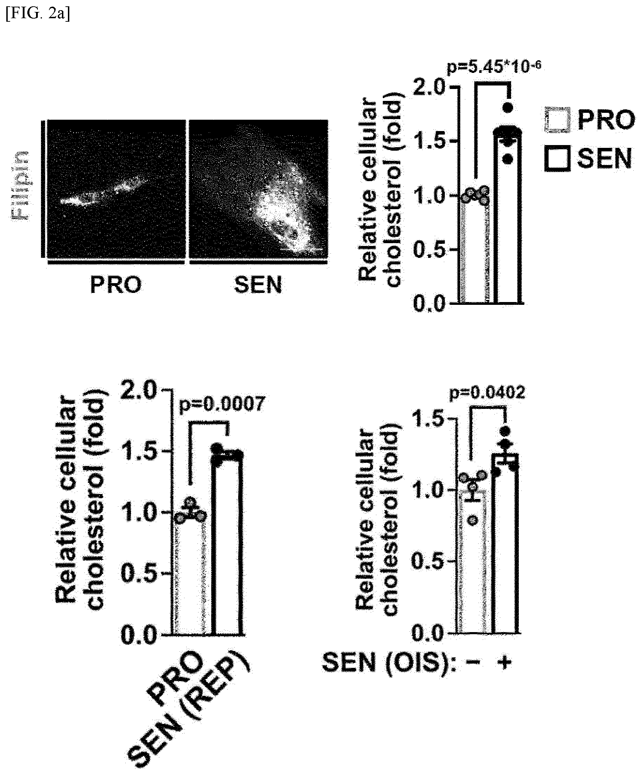

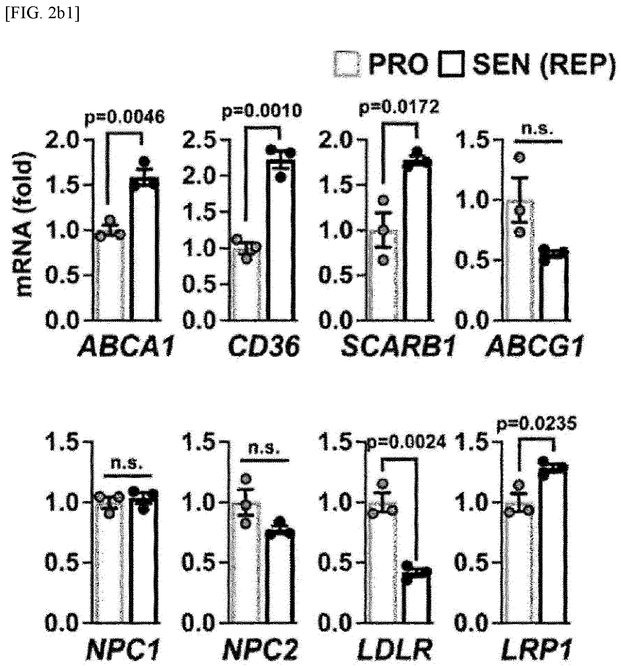

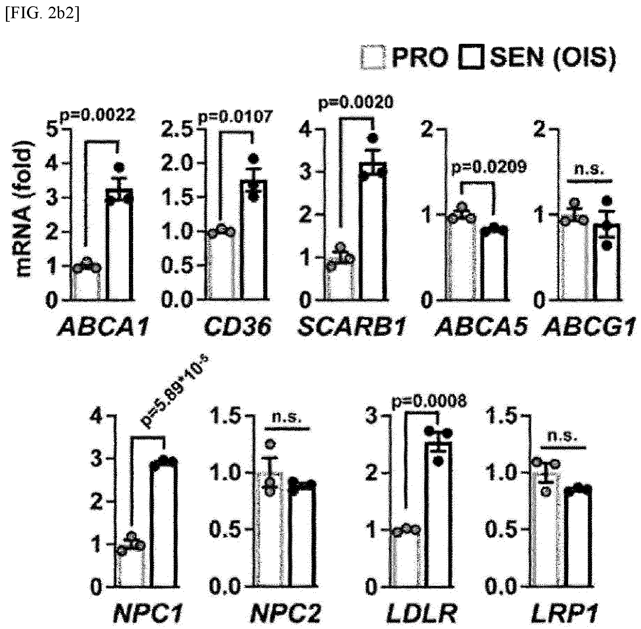

As a result of evaluation by staining analysis using Filipin and enzymatic measurement of cholesterol, cholesterol levels were found to be increased in DNA damage-induced senescent cells (SEN), replication-exhausted senescent cells (SEN (REP)), and oncogene-induced senescent cells (SEN (OIS)) compared to normal proliferating cells (PRO) (FIG. 2a). In addition, the expression of cholesterol transporter genes such as CD36, LRP1, ABCA1 and SCARB1, which are involved in cholesterol influx and efflux, was increased compared to normal proliferating cells (PRO) (FIG. 2b).

Therefore, it was confirmed that, in addition to cholesterol synthesis genes (such as HMGCR), genes involved in cholesterol influx and efflux (such as ABCA1, LRP1 and SCARB1) also play a role in changes in cholesterol metabolism during senescence.

### 1-2. Suppression of SASP factors by cholesterol depletion

To investigate the functional consequences of age-related changes in cholesterol levels, cellular cholesterol was depleted using methyl-β-cyclodextrin (MβCD), and multiple senescence markers were examined during DNA damage-induced aging, including senescence-associated β-galactosidase (SA-β-Gal) activity, cell cycle arrest, and expression of senescence-associated secretory phenotype (SASP) factors.

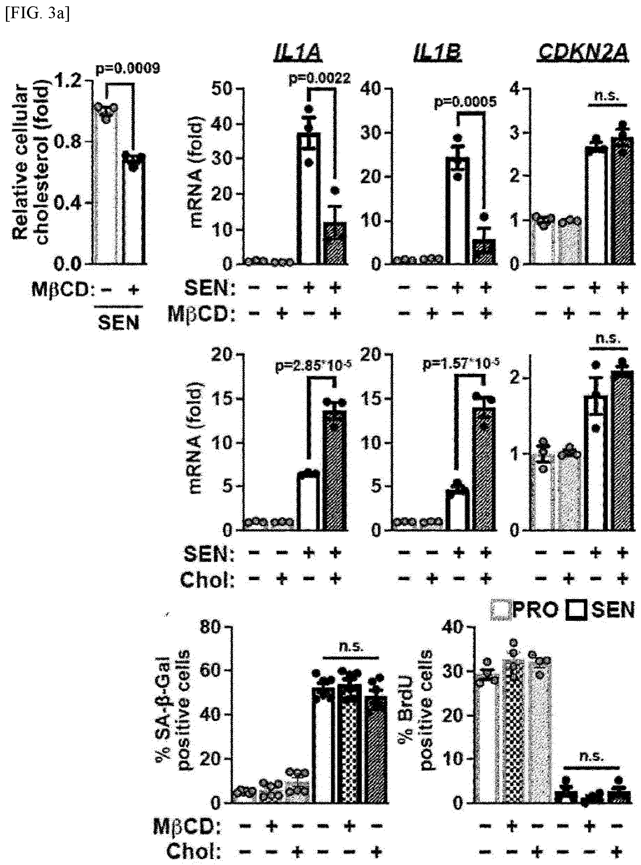

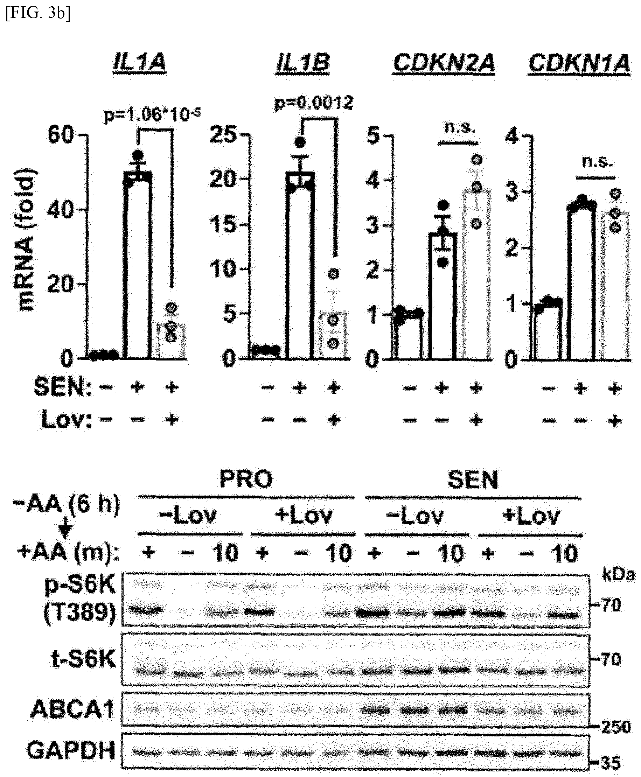

Cholesterol depletion did not affect CDKN2A expression, SA-β-Gal activity, or cell growth arrest as assessed by BrdU incorporation during senescence, but it significantly suppressed expression of SASP genes (IL1A, IL1B). In contrast, cholesterol stimulation had little effect on CDKN2A expression but significantly increased the expression of SASP genes (IL1A, IL1B) (FIG. 3a). In addition, when senescent cells were treated with lovastatin, a cholesterol inhibitor, the expression of SASP genes (IL1A, IL1B) was suppressed without affecting expression of CDKN2A or CDKN1A. Moreover, the expression of the cholesterol transporter gene ABCA1, which was activated in senescent cells compared to normal proliferating cells (PRO), was also suppressed (FIG. 3b).

These results confirm that SASP secretion in senescent cells is influenced more by cholesterol metabolism than by cell growth arrest.

### Example 2: Changes in cholesterol metabolism in senescent cells by ABCA1

### 2-1. ABCA1 expression and SASP activity

To investigate its effect on SASP expression, the cholesterol transporter gene ABCA1, which was significantly increased in senescent cells as confirmed in Example 1, was knocked out.

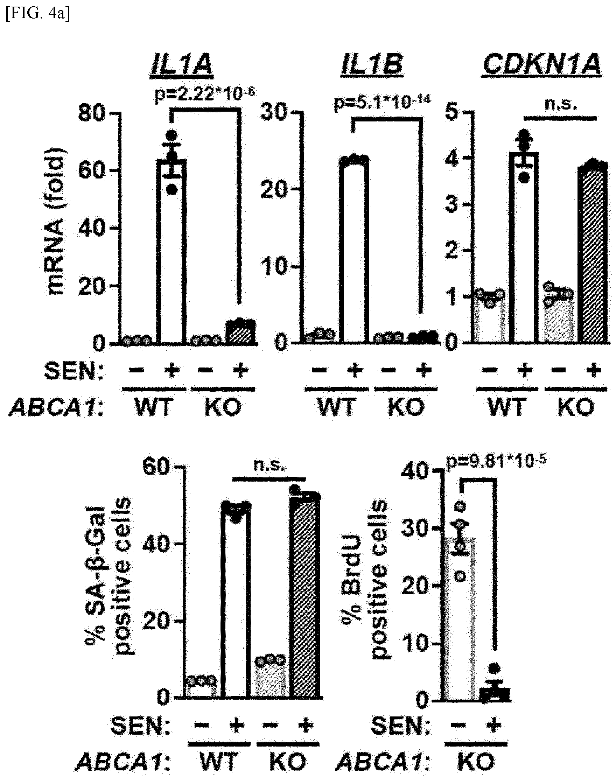

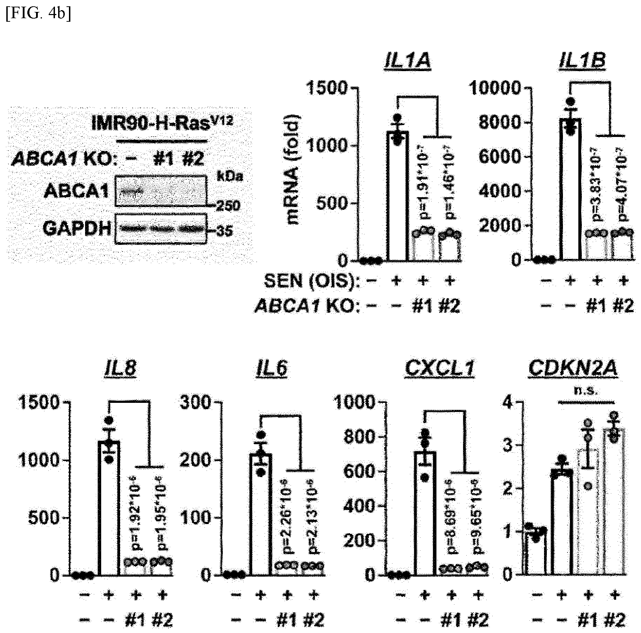

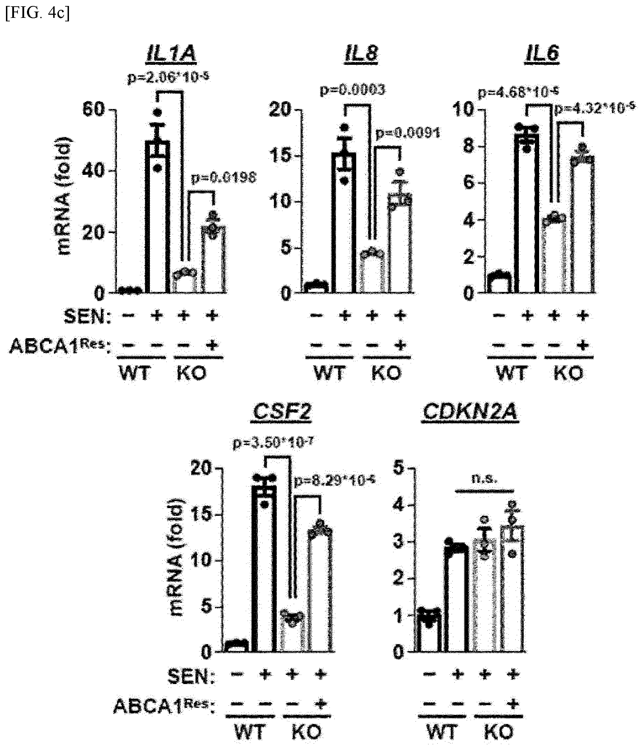

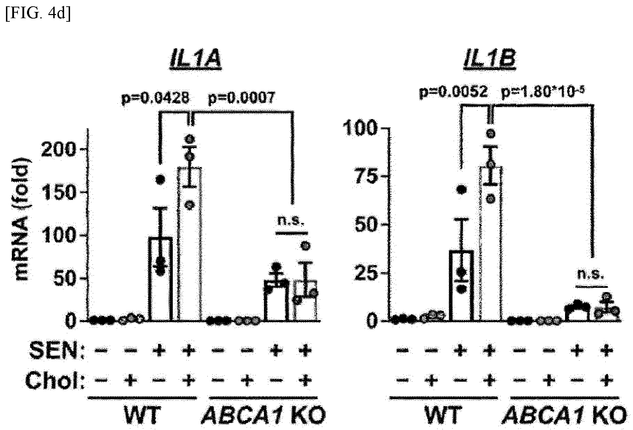

As a result, when ABCA1 was knocked out, CDKN1A expression was not affected in DNA damage-induced senescent cells (SEN), but the expression of SASP genes (IL1A, IL1B) was significantly suppressed (FIG. 4a). In oncogene-induced senescent cells (SEN (OIS)), CDKN2A expression was also unaffected, whereas the expression of SASP genes (IL1A, IL1B, IL6, CXCL1) was significantly reduced (FIG. 4b). Additionally, suppressed expression of SASP genes (IL1A, IL1B, IL6, CSF2) in ABCA1 knockout cells was restored by partial reactivation of ABCA1, with little effect on CDKN2A expression (FIG. 4c). Furthermore, cholesterol stimulation did not affect the expression levels of SASP genes in ABCA1 knockout cells (FIG. 4d).

These results indicate that changes in cholesterol metabolism during senescence are more closely associated with SASP regulation than with cell growth arrest, and that SASP factors are depended on ABCA1. It was also confirmed that changes in cholesterol metabolism during cellular senescence depend on ABCA1.

### 2-2. ABCA1 is a key factor of the GATA4-SASP pathway

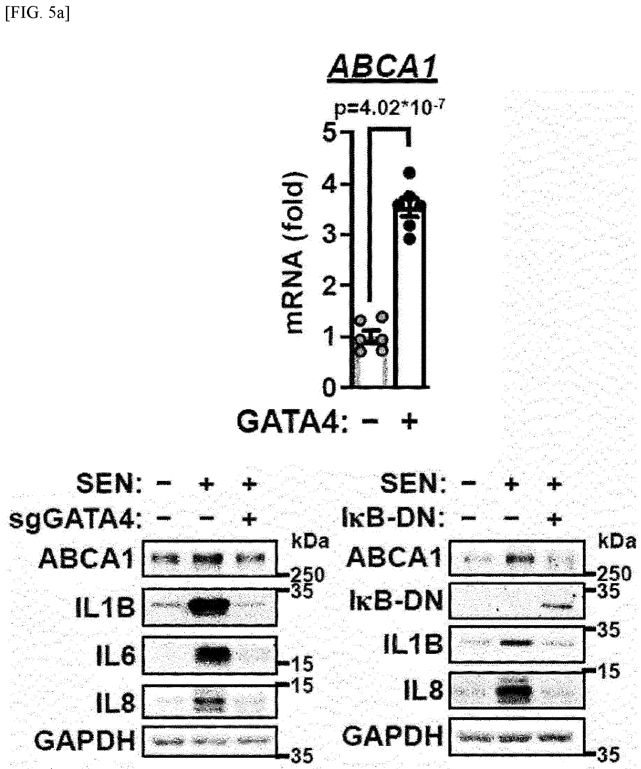

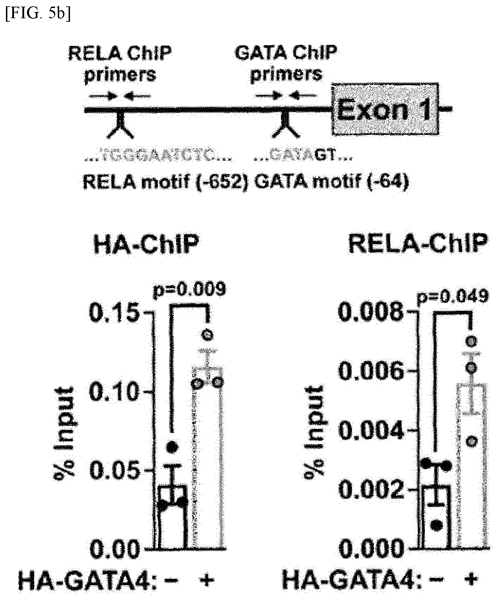

The interaction between GATA4, a key transcriptional regulator of SASP, and ABCA1 was observed. As a result, ectopic expression of GATA4 led to an increase in ABCA1 expression, and when GATA4 was deleted in DNA damage-induced senescent cells (SEN), the expression of ABCA1 and SASP factors was significantly suppressed. In addition, GATA4 initiates SASP together with the transcription factor NF-κB, and it was confirmed that inhibition of NF-κB in DNA damage-induced senescent cells (SEN) resulted in suppressed ABCA1 expression (FIG. 5a). Furthermore, it was confirmed that ectopically expressed GATA4 and endogenous RELA, a major NF-κB subunit, directly bound to the promoter region of ABCA1 (FIG. 5b).

These results confirmed that the GATA4-NF-κB pathway in the senescence-regulatory network primarily activates ABCA1, and that ABCA1 expression contributes to the activation of SASP factors in various tissues during the aging process.

### 2-3. Regulation of SASP activity by ABCA1 through the mTORC1 pathway

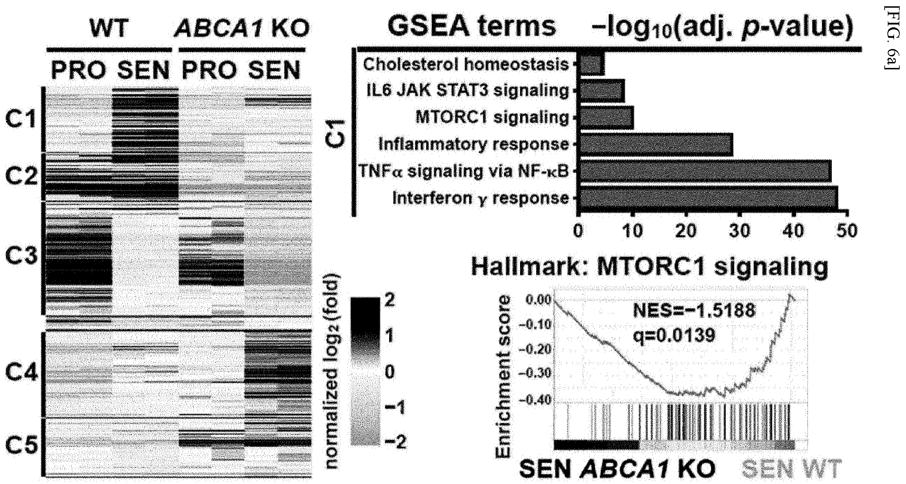

The gene set enrichment analysis (GSEA) results showed that genes (cluster 1) whose expression was decreased upon ABCA1 deletion were highly related to interferon-γ response, TNFα signaling, and inflammatory response, and were strongly associated with mTORC1 signaling (FIG. 6A). Therefore, the inventors predicted that ABCA1 would be involved in mTORC1 pathway activity during the aging process, and evaluated the phosphorylation of S6 kinase 1 (S6K1), a substrate of mTORC1, and the overall translation rate, which is a characteristic of mTORC1 activity.

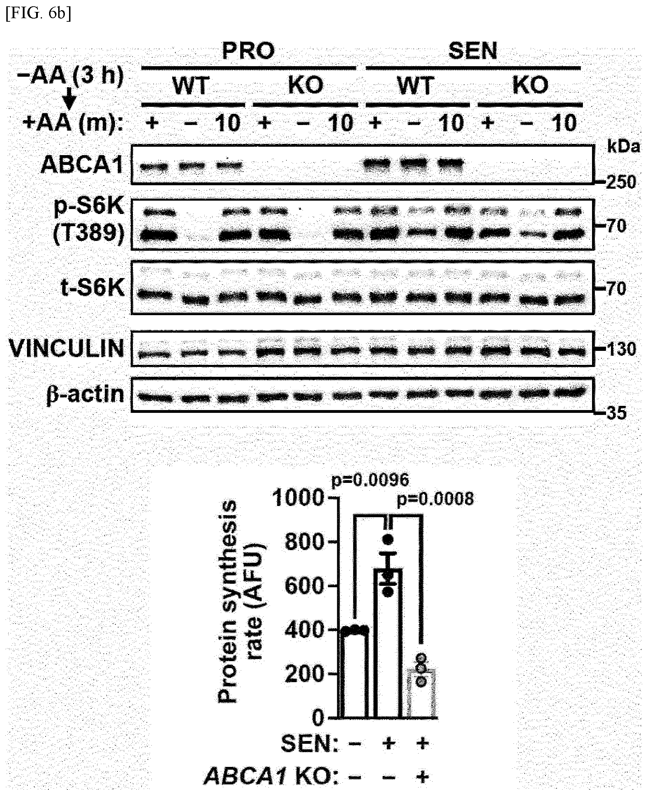

As a result, mTORC1 activity was better maintained in DNA damage-induced senescent cells (SEN) compared to normal proliferating cells (PRO), and ABCA1 knockout significantly suppressed mTORC1 activity and global translation rate (AFU) (FIG. 6b). This was similar to the result observed upon treatment with the cholesterol inhibitor lovastatin (FIG. 3b), further confirming that ABCA1 is involved in mTORC1 pathway activity.

mTORC1 regulates cell growth and proliferation upon activation at lysosomes, and it was predicted that higher mTORC1 activity maintained by ABCA1 in senescent cells would stimulate the expression of SASP factors at the transcriptional level.

### Example 3: In vitro identification of the lysosomal ABCA1-lysosomal cholesterol-mTORC1/SASP axis

### 3-1. Lysosomal cholesterol accumulation mediated by lysosomal ABCA1

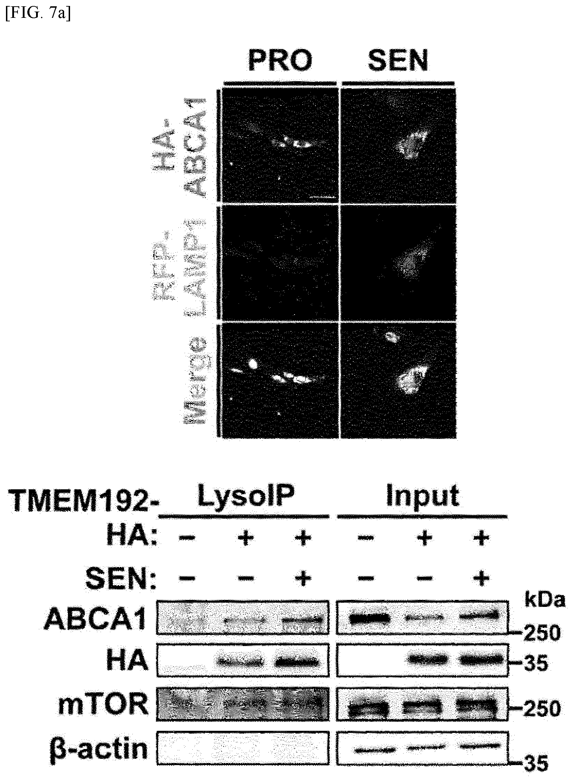

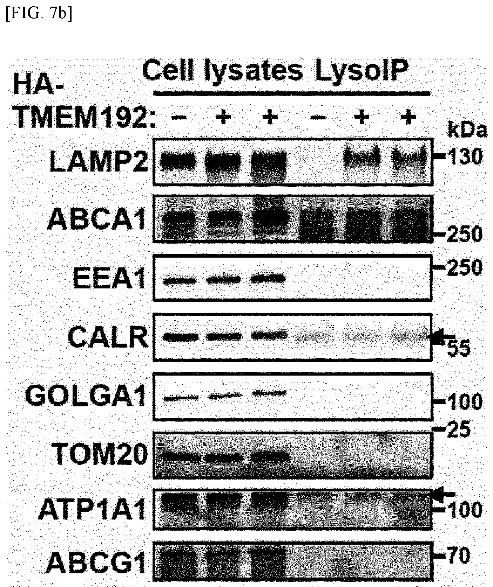

It was analyzed how ABCA1, which mediates cholesterol transport at the plasma membrane, is involved in cholesterol accumulation. As a result of evaluation by immunofluorescence analysis, it was confirmed that a subset of ABCA1 was localized to lysosomes upon ectopic expression (FIG. 7a). In addition, immunoblotting analysis of lysosome immunoprecipitates (LysoIP) showed that endogenous ABCA1 was abundantly present in lysosomes, unlike ABCG1, another member of the ABC transporter family that mediates cholesterol efflux (FIG. 7b). This confirmed the intracellular localization of ABCA1.

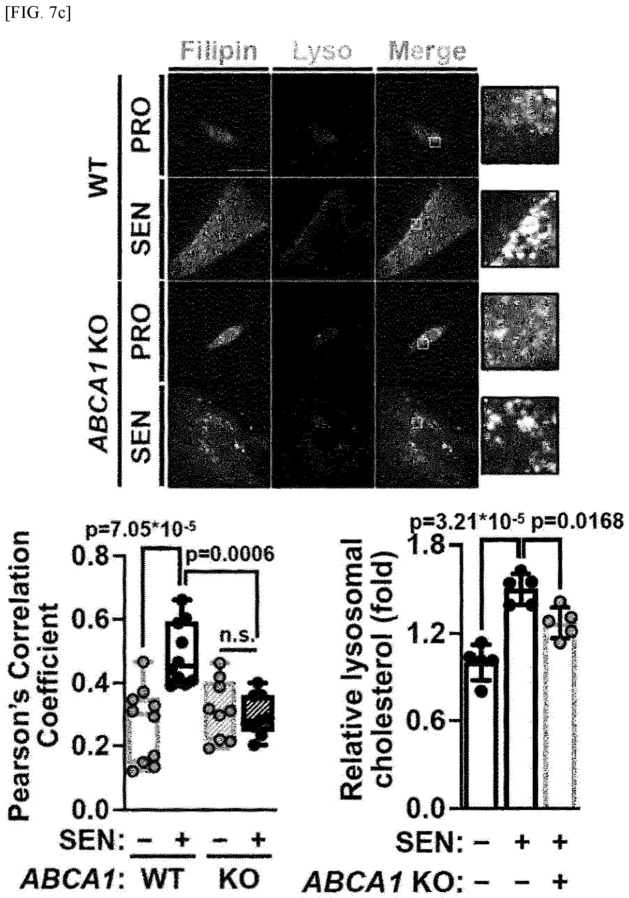

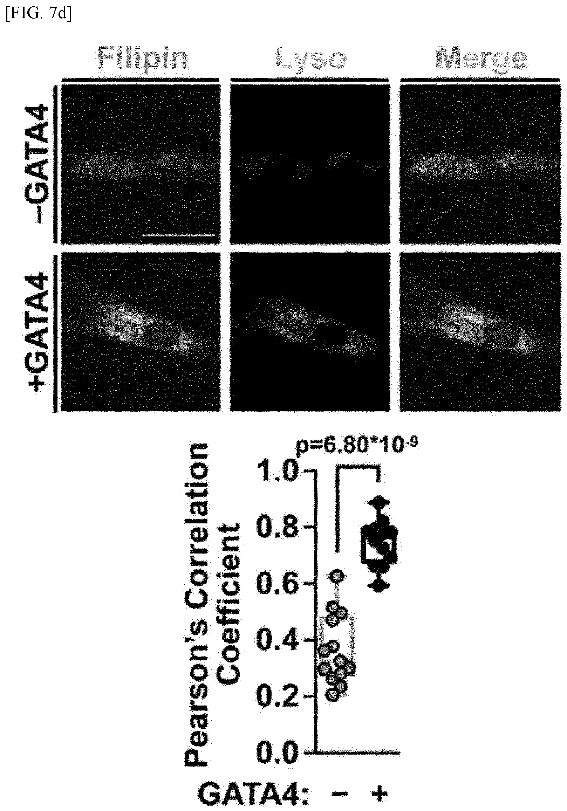

In addition, Filipin staining analysis of isolated lysosomes and enzymatic measurement of cholesterol revealed that lysosomal cholesterol accumulation was reduced in senescent cells lacking ABCA1 (FIG. 7c). In contrast, ectopic expression of GATA4, which induces ABCA1 expression, increased lysosomal cholesterol accumulation (FIG. 7d).

These results suggest that the increased lysosomal cholesterol accumulation in senescent cells may be due to the lysosomal localization of ABCA1. Additional experiments were performed to confirm this.

### 3-3. Activation of mTORC1 and SASP by lysosomal ABCA1

To specifically confirm the effect of increased expression of lysosomal ABCA1 on SASP activity, experiments were conducted using probucol, an ABCA1 modulator that has been reported to limit its localization to intracellular compartments including lysosomes, and glyburide, a broad-spectrum inhibitor of the ABC transport protein family that promotes channel closure of ABC transporters. The prepared senescent cell models were treated with probucol (10 µM) and/or glyburide (75 µM), and immunoblotting analysis was performed on lysosome immunoprecipitates (LysoIP).

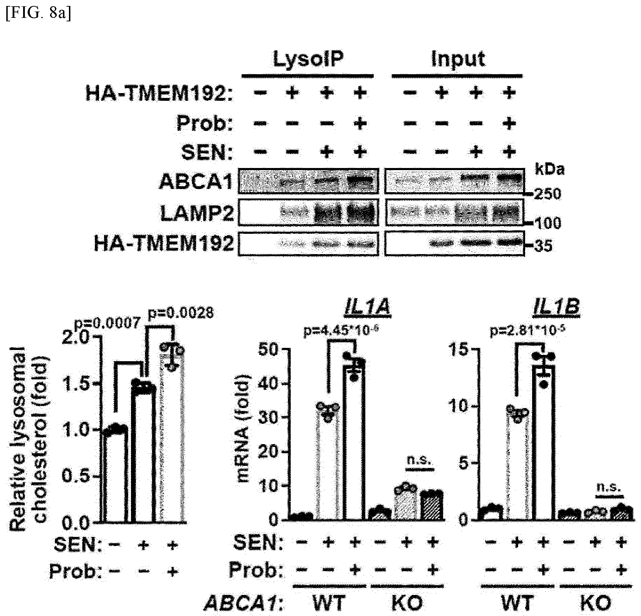

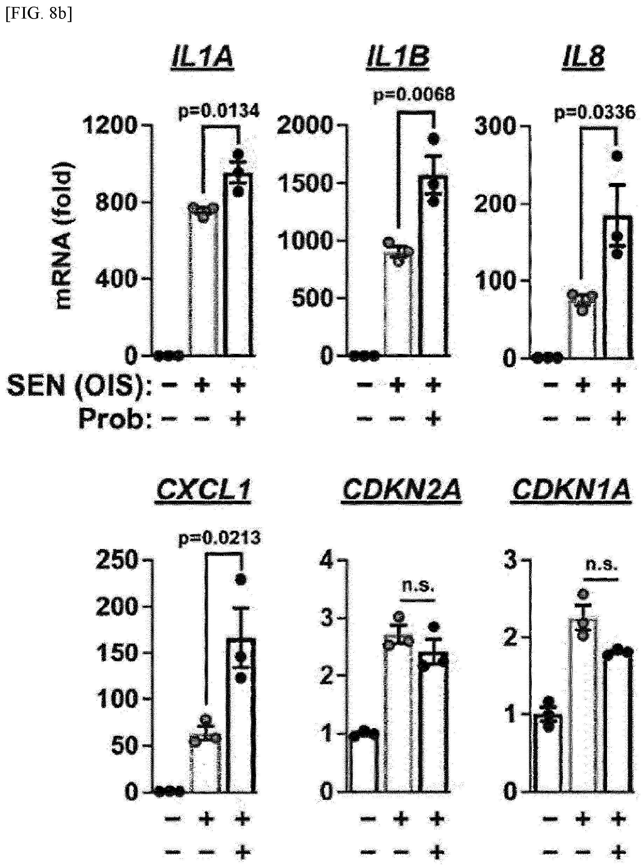

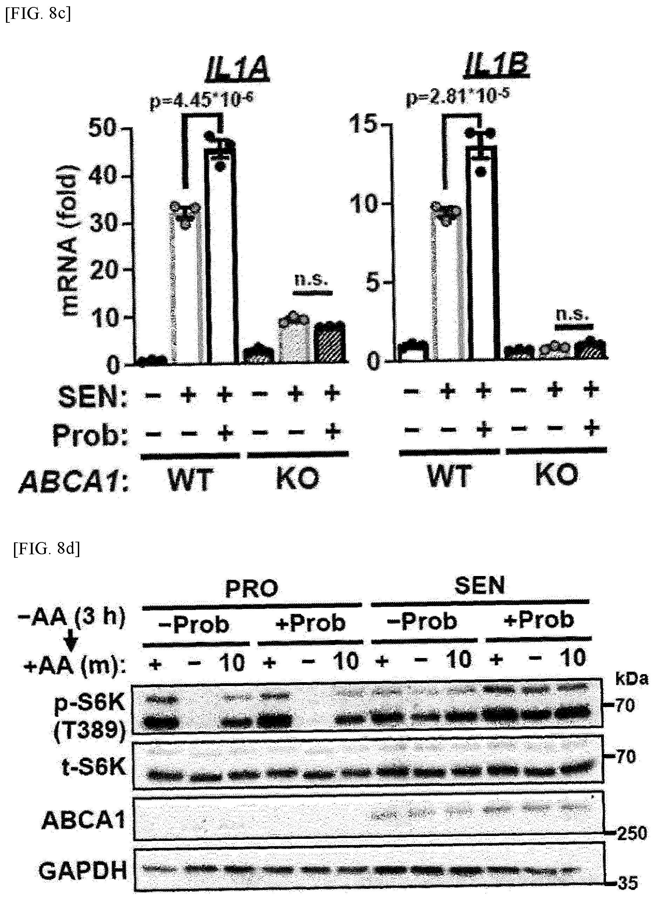

As a result of probucol treatment, the increased expression of ABCA1 in DNA damage-induced senescent cells (SEN) was further enhanced, and probucol treatment promoted cholesterol accumulation (FIG. 8a). SASP activity was also increased by probucol treatment in oncogene-induced senescent cells (SEN (OIS)) (FIG. 8b). However, mTORC1 and SASP (IL1A, IL1B) were not activated by probucol treatment in DNA damage-induced senescent cells (SEN) lacking ABCA1 (FIG. 8c). This confirms that the SASP-promoting effect of probucol depends on ABCA1.

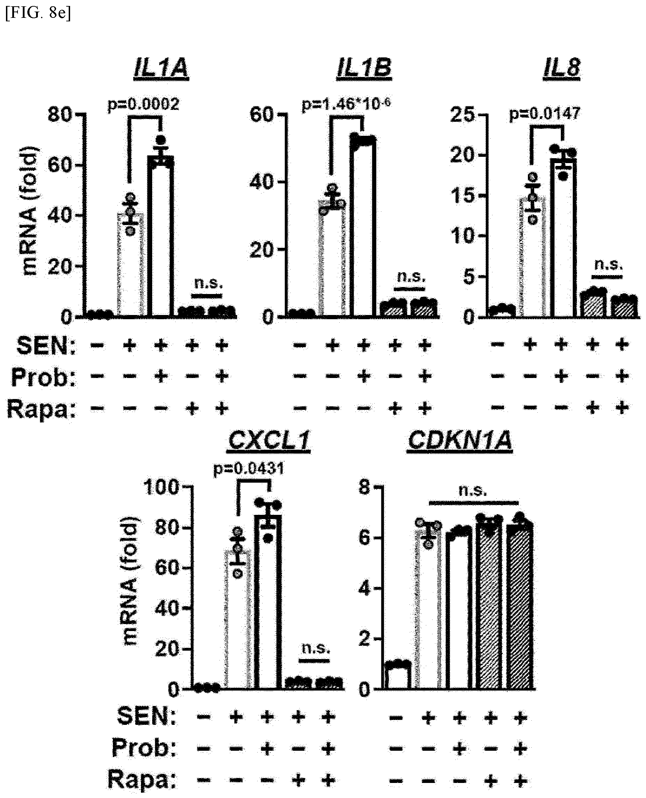

In addition, SASP activation following probucol treatment was dependent on mTORC1 activation (FIG. 8d), and SASP activation by probucol was completely inhibited by rapamycin, which was used to pharmacologically inhibit mTORC1 (FIG. 8e), confirming that SASP factors were regulated via mTORC1 signaling.

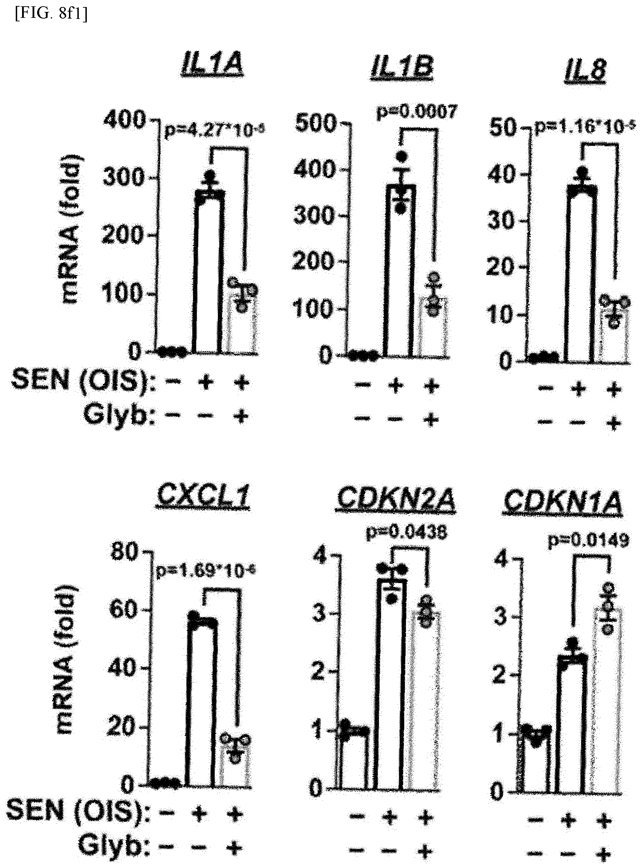

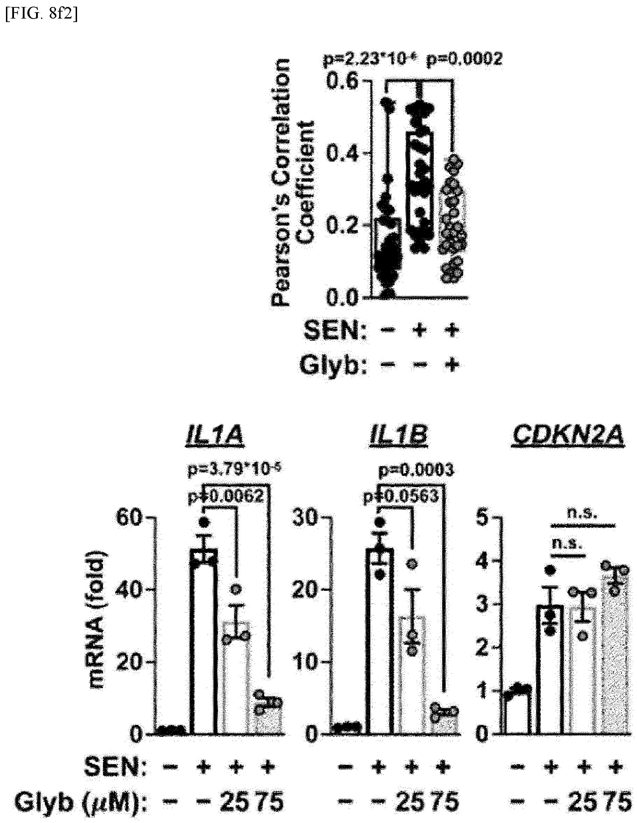

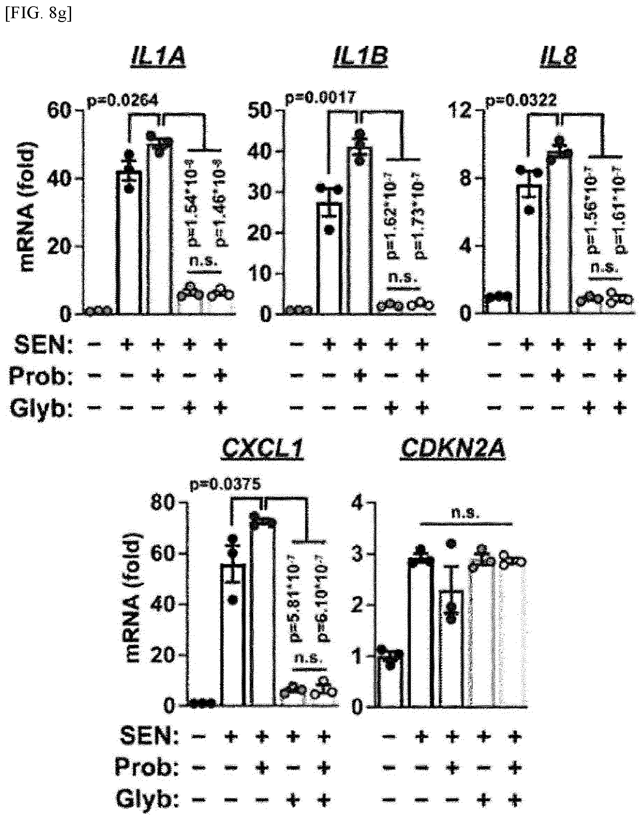

As a result of glyburide treatment, in contrast to probucol, lysosomal cholesterol accumulation and SASP activity were inhibited in DNA damage-induced senescent cells (SEN) and oncogene-induced senescent cells (SEN (OIS)) (FIG. 8f). In addition, when glyburide was applied to senescence-induced cells treated with probucol, it was confirmed that the expression of SASP factors activated by probucol was completely suppressed (FIG. 8g).

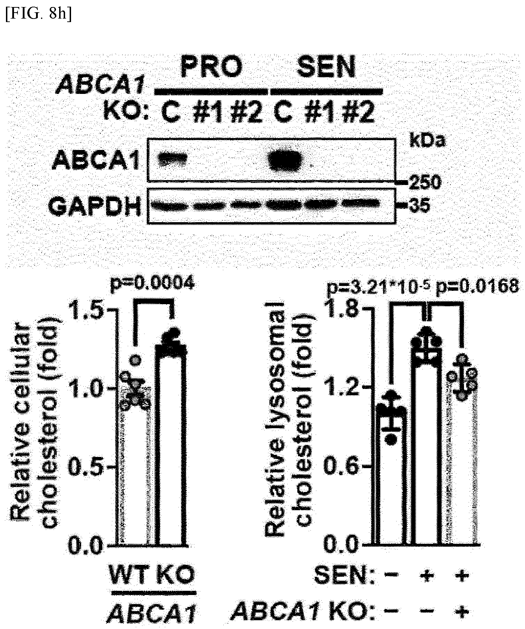

Meanwhile, referring to FIG. 8h, genetic deletion of ABCA1 increased the total cellular cholesterol level due to inhibition of cholesterol efflux by ABCA1 (FIG. 8h1). However, since ABCA1 localized to lysosomes was also suppressed, lysosomal cholesterol levels were instead decreased (FIG. 8h2). This result confirms that lysosomal ABCA1 functions as a lysosomal cholesterol transporter. In addition, as shown in FIGS. 4b and FIGS. 6, deletion of ABCA1 suppressed the activation of mTORC1 and SASP in senescence-induced cells.

Taken together, these results suggest that lysosomal cholesterol, rather than total cellular cholesterol, is more directly involved in mTORC1 activation and SASP-induced age-related inflammation. Further, lysosomal cholesterol accumulation was shown to result from the lysosomal localization of ABCA1. This suggests that ABCA1 is not merely a cellular cholesterol transporter that regulates total cholesterol levels, but also a key regulator of mTORC1 and SASP during senescence through its modulation of lysosomal cholesterol.

### 3-4. Lysosomal microdomain formation by lysosomal ABCA1

To determine whether lysosomal cholesterol accumulation by lysosomal ABCA1 contributes to the formation of microdomains, which are key components of the plasma membrane (PM) involved in membrane transport, signaling, lipid metabolism, or endocytosis, and to the activation of mTORC1, lysosomes were immunopurified and then subjected to density gradient ultracentrifugation (LysoIP-DEG) to observe the formation of lysosomal microdomains during senescence and upon ABCA1 deletion.

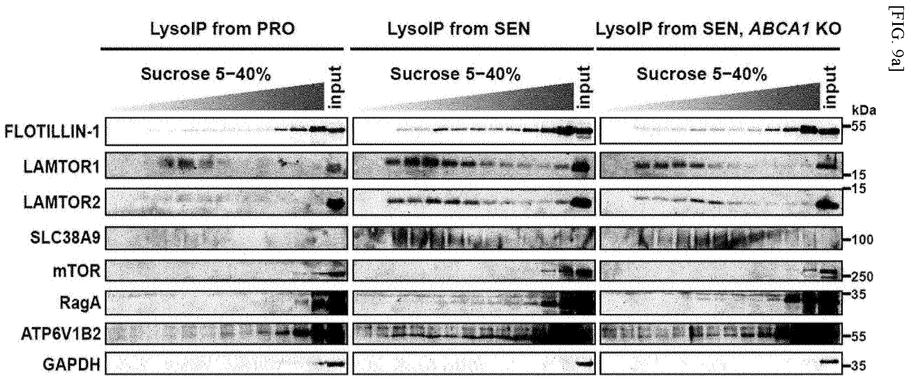

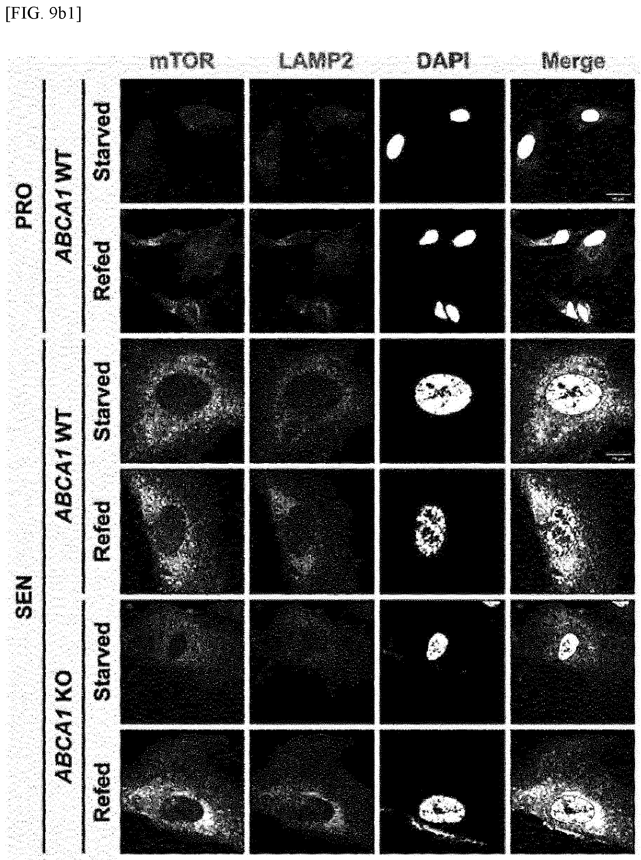

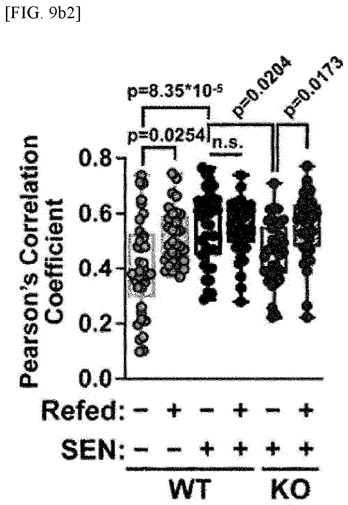

As a result, compared with the lysosomes of normal proliferating cells (PRO), microdomains positive for FLOTILLIN-1, a marker of detergent-resistant microdomains, were increased in the lysosomes of senescent cells (SEN), and the mTORC1 scaffolding complex, including Ragulator, SLC38A9 and V-ATPase, was activated. In addition, ABCA1 deletion in senescent cells significantly reduced the formation of lysosomal microdomains and the activation of the mTORC1 scaffolding complex (FIG. 9a), and also reduced the sustained lysosomal localization of mTOR, as assessed by immunofluorescence analysis (FIG. 9b)

These results confirmed that lysosomal microdomains in senescent cells sustain high mTORC1 activity, and that ABCA1 plays a critical role in forming lysosomal microdomains that support the mTORC1 scaffolding complex and promote mTORC1 activation.

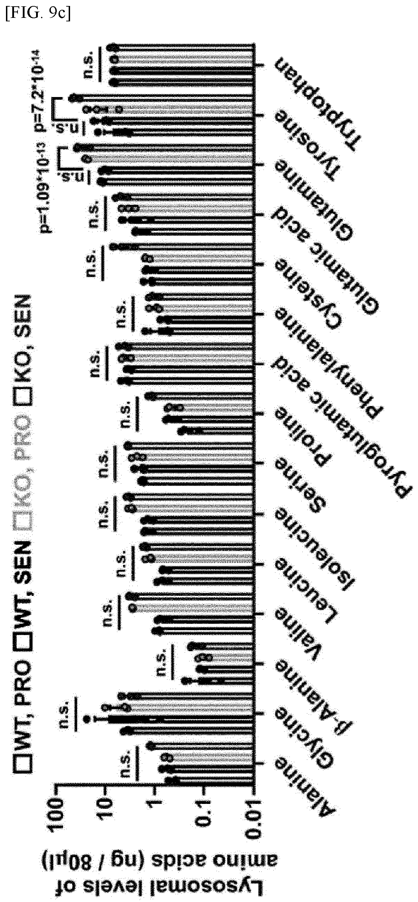

Meanwhile, the level of lysosomal amino acids, another important nutrient source for mTORC1 in relation to lysosomal cholesterol metabolism, did not change during senescence and was not significantly affected by ABCA1 deletion (FIG. 9c). Therefore, it was confirmed that lysosomal cholesterol accumulated by lysosomal ABCA1 induces microdomains that activate mTORC1 through the local clustering of mTORC1 scaffolding complexes.

### Example 4: In vivo validation of the lysosomal ABCA1-lysosomal cholesterol-mTORC1/SASP axis

### 4-1. Transcriptome profiling of human osteoarthritis tissue

To perform transcriptional profiling in osteoarthritis tissue, one of the pathological conditions associated with aging, GSEA was conducted using data from GSE57218 (human OA transcriptome) and GSE64553 (MRC5 replicative senescence transcriptome). The gene sets for mTORC1 signaling (total 200 genes) and cholesterol homeostasis (total 200 genes) were obtained from the Molecular Signature Database (MSigDB).

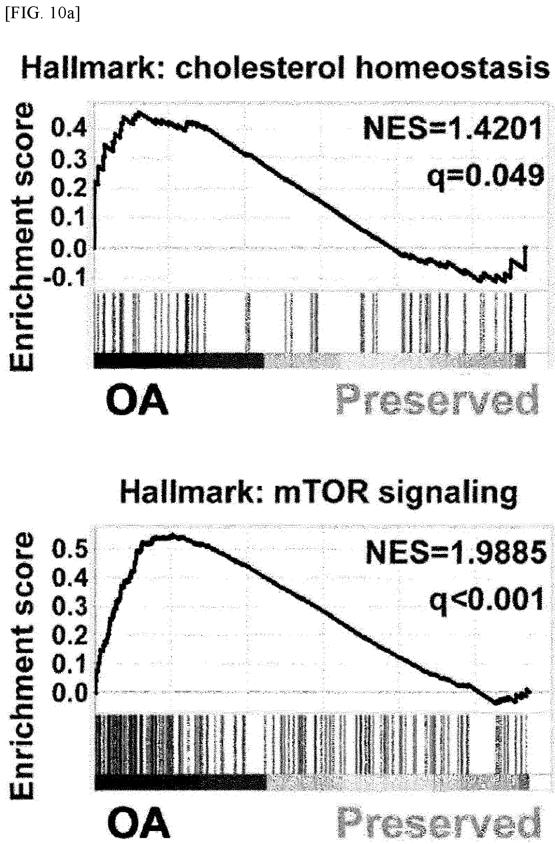

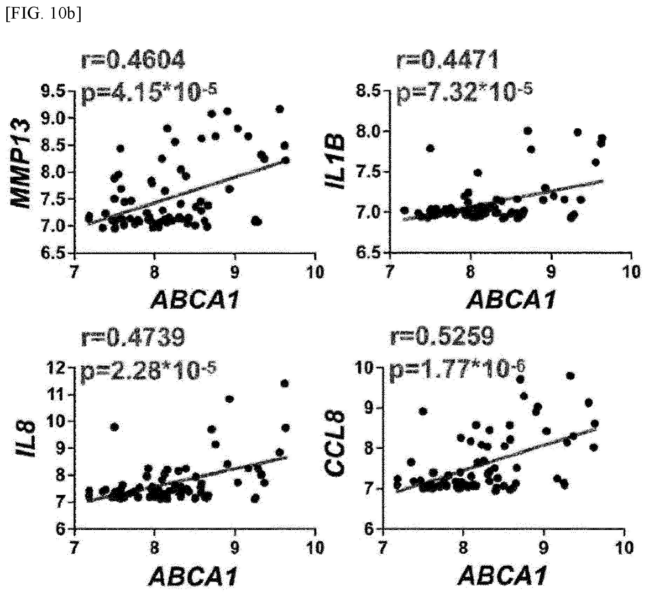

As a result of the GSEA, it was confirmed that the enrichment scores of cholesterol homeostasis and mTORC1 signaling signatures were significantly upregulated in the genes differentially expressed during the progression of osteoarthritis (FIG. 10a). In addition, ABCA1 expression was found to be positively correlated with the expression of several SASP genes, including MMP13, IL1B, IL8 and CCL8 (FIG. 10b).

### 4-2. Mouse primary cultured chondrocytes

Senescence of chondrocytes promotes age-related musculoskeletal disorders. The correlation between cholesterol metabolism and ABCA1 was analyzed in a DNA damage-induced aging model (SEN) of mouse primary cultured chondrocytes isolated from the femoral condyles and tibial plateaus of 5-day-old ICR mice.

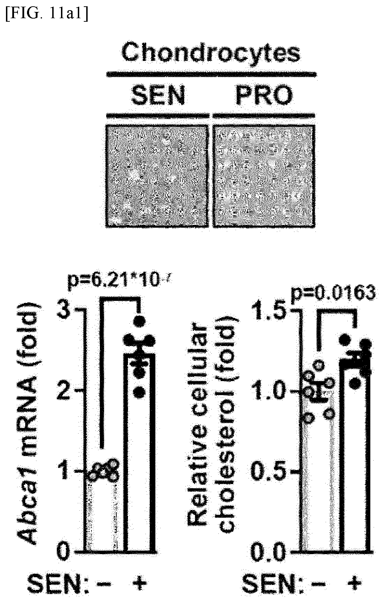

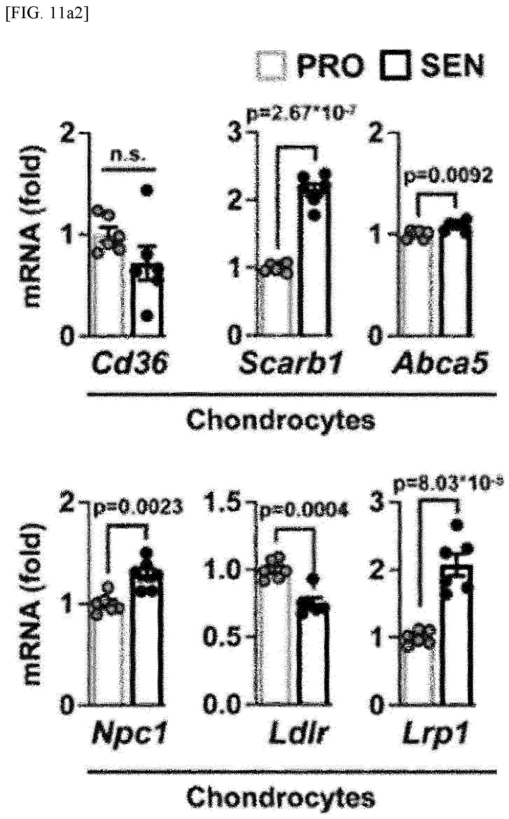

As a result, genes such as Scarb1 and Abca1, which are involved in cholesterol influx and efflux during chondrocyte aging, were significantly upregulated, and the cellular cholesterol level was also increased (FIG. 11a).

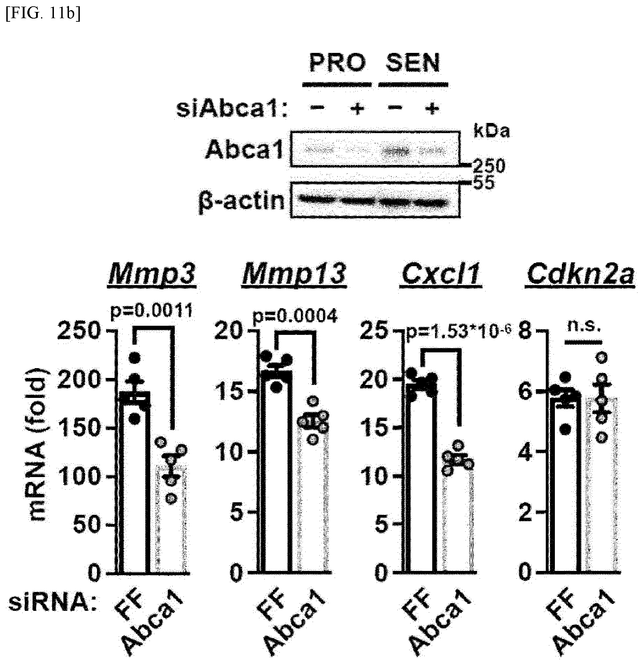

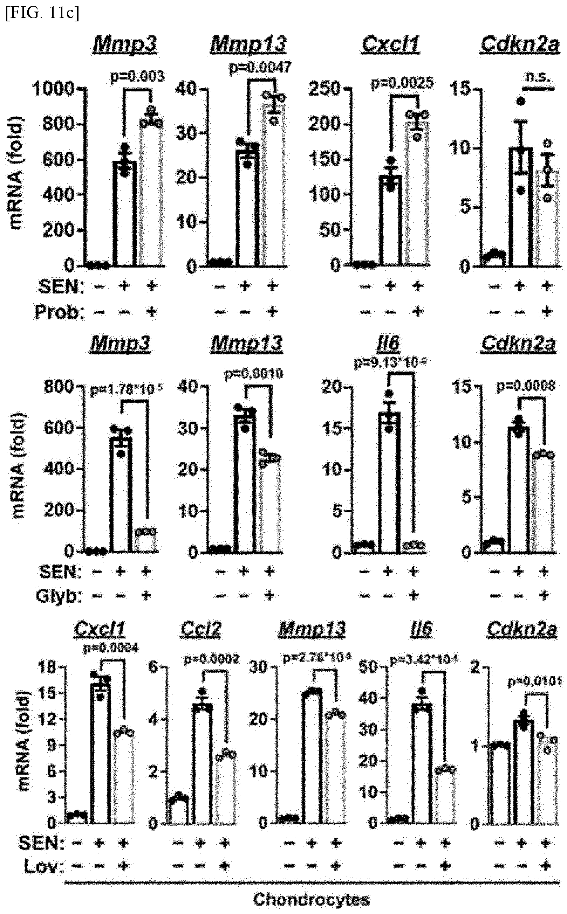

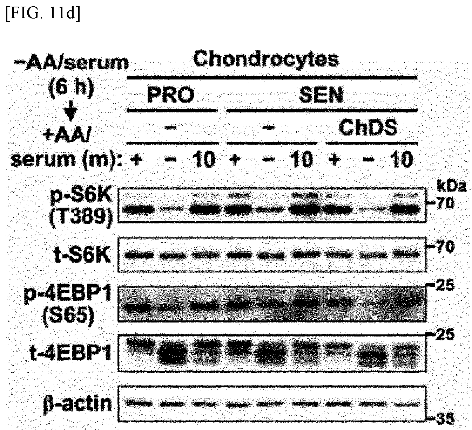

When ABCA1 was depleted in mouse primary cultured chondrocytes (SEN), the expression of SASP genes, including Mmp3, Mmp13 and Cxcl1, was suppressed, while the expression of Cdkn2a remained unchanged (FIG. 11b). In addition, SASP factors were upregulated by treatment with probucol (10 µM), whereas these SASP factors were suppressed by treatment with glyburide (75 µM) or lovastatin (2 µM) (FIG. 11c). Induction of cholesterol depletion inhibited the sustained activation pattern of mTORC1 (FIG. 11d), which was consistent with the results from the senescent cell model (*in vitro*) in Example 1. Therefore, it was confirmed that the lysosomal ABCA1 gene is a major factor in SASP regulation *in vivo.*

### Example 5: Alleviation of osteoarthritis progression through in vivo modulation of the ABCA1-lysosomal cholesterol-mTORC1/SASP axis

To investigate the role of lysosomal cholesterol partitioning regulated by ABCA1 in age-related inflammation *in vivo,* ABCA1 modulators were administered to male mice subjected to DMM surgery, and their effects on the progression of post-traumatic osteoarthritis were observed.

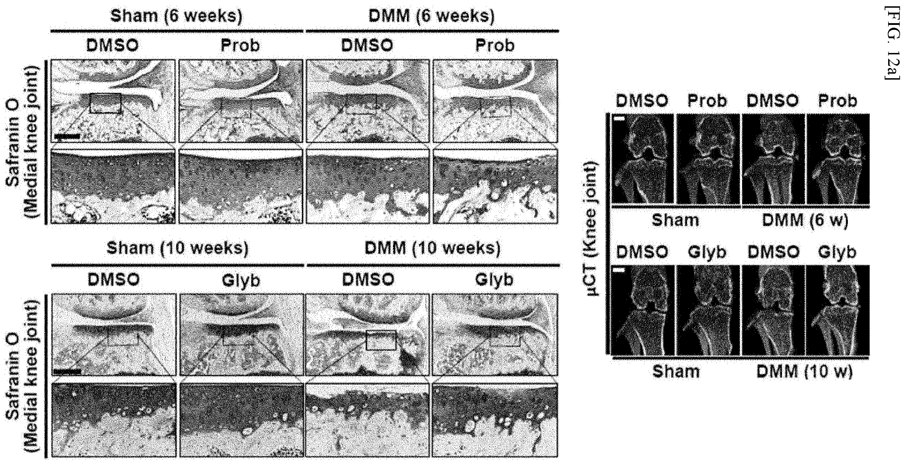

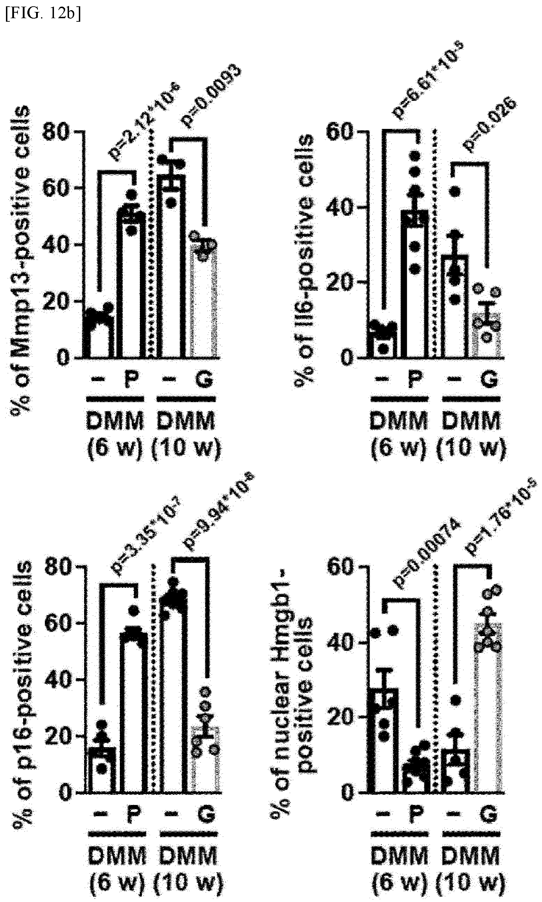

Male mice subjected to either DMM surgery or sham surgery were intra-articularly injected with 10 µl of probucol (200 µM) dissolved in DMSO once a week for 6 weeks, or with 10 µl of PBS-suspended hydrogel containing 3 mM glyburide (PBS:hydrogel = 1:1) every 10 days for 10 weeks. After the treatment period, the mice were sacrificed. Histological assessment of cartilage destruction in the knee joints using Safranin O staining revealed that intra-articular injection of probucol worsened osteoarthritis progression, inducing cartilage loss and early onset of subchondral sclerosis in the medial compartment of the knee joint. In contrast, glyburide administration alleviated cartilage degradation (FIG. 12a). Furthermore, the expression levels of SASP factors including IL6 and MMP13, and senescence markers such as p16 and nuclear HMGB1, were increased in the probucol-treated group, but were decreased in the glyburide-treated group (FIG. 12b).

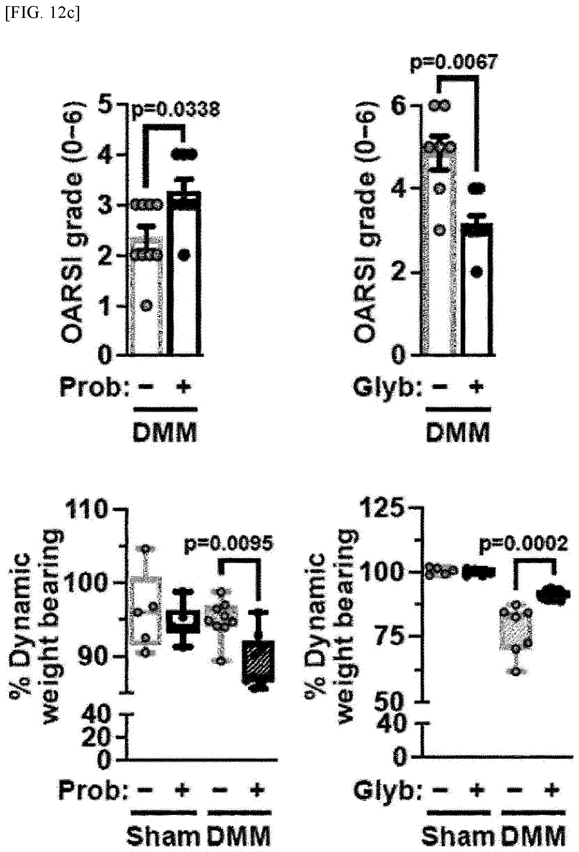

In addition, the probucol-treated group exhibited worsened osteoarthritis-related pain symptoms, as evidenced by higher Osteoarthritis Research Society International (OARSI) grades, impaired dynamic and static weight-bearing capacity, and reduced hindlimb withdrawal threshold. Conversely, the glyburide-treated group showed amelioration of these pain indicators (FIG. 12c).

Collectively, these results demonstrate that probucol promotes lysosomal localization of ABCA1, leading to lysosomal cholesterol accumulation and exacerbation of osteoarthritis progression and pain. In contrast, glyburide suppresses lysosomal ABCA1, thereby reducing lysosomal cholesterol accumulation and alleviating osteoarthritis progression and associated pain.

### Example 6: Age-dependent ABCA1 expression and SASP activity in various senescent tissues

To evaluate whether the ABCA1-lysosomal cholesterol-mTORC1/SASP axis, whose targeting has been shown to suppress age-related inflammation and improve therapeutic efficacy of osteoarthritis in chondrocytes, is reflected in various aging human tissues, an analysis was conducted using the Genotype-Tissue Expression (GTEx) dataset.

The GTEx dataset, which includes gene expression profiles from various disease-free human tissues across different age groups, was categorized into two age groups: "young" (20 - 39 years) and "elderly" (60 - 79 years). SASP signature scores were then calculated for each sample, and their correlation with ABCA1 expression was analyzed.

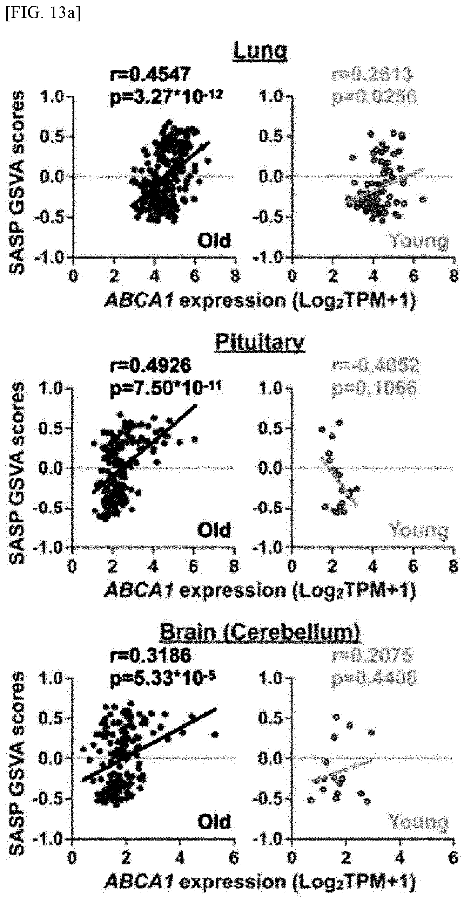

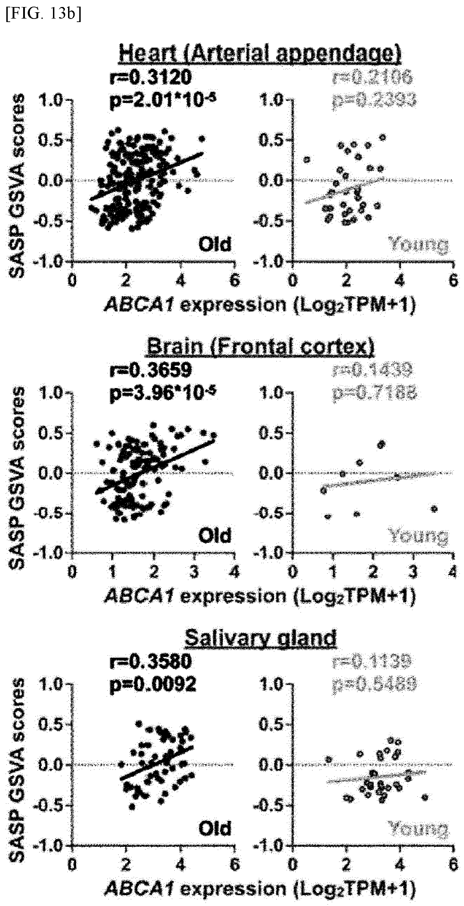

As a result, a positive correlation between SASP signature scores and ABCA1 expression was observed in various tissues of the elderly, including the brain and lungs, whereas this correlation was minimal or absent in the younger group (FIG. 13). These findings suggest that targeting the ABCA1-lysosomal cholesterol-mTORC1/SASP axis may effectively suppress age-related inflammation in senescent tissues in an age-dependent manner, and thus may hold therapeutic and diagnostic potential for various aging-related diseases.

## Claims

1. A pharmaceutical composition for use in preventing or treating of age-related musculoskeletal disorders, comprising a lysosomal ABCA1 inhibitor.

2. The pharmaceutical composition for use in preventing or treating of age-related musculoskeletal disorders according to claim 1, wherein the lysosomal ABCA1 inhibitor comprises at least one selected from the group consisting of gRNA, siRNA, shRNA, dsRNA, miRNA, PAN, ribozyme, an antisense nucleic acid, a peptide, a protein, an aptamer, an antibody and a compound.

3. The pharmaceutical composition for use in preventing or treating of age-related musculoskeletal disorders according to claim 2, wherein the gRNA comprises at least one nucleotide sequence selected from the group consisting of SEQ ID NOs: 1 to 4, and the siRNA comprises at least one nucleotide sequence selected from the group consisting of SEQ ID NOs: 5 to 8.

4. The pharmaceutical composition for use in preventing or treating of age-related musculoskeletal disorders according to claim 1, wherein the lysosomal ABCA1 inhibitor is glyburide.

5. The pharmaceutical composition for use in preventing or treating of age-related musculoskeletal disorders according to claim 1, wherein the lysosomal ABCA1 inhibitor inhibits lysosomal cholesterol accumulation in cells.

6. The pharmaceutical composition for use in preventing or treating of age-related musculoskeletal disorders according to claim 1, wherein the age-related musculoskeletal disorder is osteoarthritis or sarcopenia.

7. A food composition for use in preventing or improving of age-related musculoskeletal disorders, comprising a lysosomal ABCA1 inhibitor.

8. The food composition for use in preventing or improving of age-related musculoskeletal disorders according to claim 7, wherein the lysosomal ABCA1 inhibitor comprises at least one selected from the group consisting of gRNA, siRNA, shRNA, dsRNA, miRNA, PAN, ribozyme, an antisense nucleic acid, a peptide, a protein, an aptamer, an antibody and a compound.

9. The food composition for use in preventing or improving of age-related musculoskeletal disorders according to claim 8, wherein the gRNA comprises at least one nucleotide sequence selected from the group consisting of SEQ ID NOs: 1 to 4, and the siRNA comprises at least one nucleotide sequence selected from the group consisting of SEQ ID NOs: 5 to 8.

10. The food composition for use in preventing or improving of age-related musculoskeletal disorders according to claim 7, wherein the lysosomal ABCA1 inhibitor is glyburide.

11. A method for screening a therapeutic agent for age-related musculoskeletal disorders, comprising treating isolated cells with a candidate substance to determine whether the activity of lysosomal ABCA1 is inhibited.

12. The method for screening a therapeutic agent for age-related musculoskeletal disorders according to claim 11, wherein the cells are chondrocytes, fibroblasts or muscle cells.

13. The method for screening a therapeutic agent for age-related musculoskeletal disorders according to claim 11, wherein the age-related musculoskeletal disorder is osteoarthritis or sarcopenia.

14. A composition for use in diagnosing of age-related musculoskeletal disorders, the composition comprising an agent that specifically binds to lysosomal ABCA1 mRNA or a protein encoded thereby.

15. The composition for use in diagnosing of age-related musculoskeletal disorders according to claim 14, wherein the agent is at least one selected from the group consisting of a primer, a probe, an antisense nucleic acid, a peptide, a protein, an aptamer, an antibody and a compound.

16. The composition for use in diagnosing of age-related musculoskeletal disorders according to claim 14, wherein the age-related musculoskeletal disorder is osteoarthritis or sarcopenia.
